# EUROPEAN PATENT APPLICATION

(11) **EP 4 491 237 A2**
(43) Date of publication of application: **15.01.2025**
(21) Application number: 24201473.6
(22) Date of filing: 27.10.2017
(51) Int. Cl.: A61P 43/00

(54) **METHODS OF TREATING UROTHELIAL CARCINOMA USING AN ANTI-PD-1 ANTIBODY**

(30) Priority: 28.10.2016 US 201662414287 P
(62) Divisional of application: 17804993.8
(71) Applicant: Bristol-Myers Squibb Company, Princeton, NJ 08543 (US)
(72) Inventor: TSCHAIKA, Marina, Princeton, NJ 08543 (US)
(74) Representative: Mewburn Ellis LLP

(57) **Abstract**

This disclosure provides a method for treating a subject afflicted with a urothelial carcinoma or cancer derived therefrom, which method comprises administering to the subject an antibody or an antigen-binding portion thereof that specifically binds to a Programmed Death-1 (PD-1) receptor and inhibits PD-1 activity or the combination of (a) an antibody or an antigen-binding portion thereof that specifically binds to a PD-1 receptor and inhibits PD-1 activity; and (b) an antibody or an antigen-binding portion thereof that specifically binds to a Cytotoxic T-Lymphocyte Antigen-4 (CTLA-4) and inhibits CTLA-4 activity.

## Description

### CROSS-REFERENCE TO RELATED APPLICATIONS

This application claims the benefit of U.S. Provisional Application Serial No. 62/414,287 filed October 28, 2016, which is incorporated herein by reference in its entirety.

### FIELD OF THE INVENTION

This invention relates to methods for treating a urothelial carcinoma or cancer derived therefrom in a subject comprising administering to the subject an anti-Programmed Death-1 (PD-1) antibody or a combination of an anti-PD-1 antibody and an anti-Cytotoxic T-Lymphocyte Antigen-4 (CTLA-4) antibody.

### BACKGROUND OF THE INVENTION

Human cancers harbor numerous genetic and epigenetic alterations, generating neoantigens potentially recognizable by the immune system (Sjoblom et al. (2006) Science 314:268-74). The adaptive immune system, comprised of T and B lymphocytes, has powerful anti-cancer potential, with a broad capacity and exquisite specificity to respond to diverse tumor antigens. Further, the immune system demonstrates considerable plasticity and a memory component. The successful harnessing of all these attributes of the adaptive immune system would make immunotherapy unique among all cancer treatment modalities.

PD-1 is a key immune checkpoint receptor expressed by activated T and B cells and mediates immunosuppression. PD-1 is a member of the CD28 family of receptors, which includes CD28, CTLA-4, ICOS, PD-1, and BTLA. Two cell surface glycoprotein ligands for PD-1 have been identified, Programmed Death Ligand-1 (PD-L1) and Programmed Death Ligand-2 (PD-L2), that are expressed on antigen-presenting cells as well as many human cancers and have been shown to down-regulate T cell activation and cytokine secretion upon binding to PD-1.

Nivolumab (formerly designated 5C4, BMS-936558, MDX-1106, or ONO-4538) is a fully human IgG4 (S228P) PD-1 immune checkpoint inhibitor antibody that selectively prevents interaction with PD-1 ligands (PD-L1 and PD-L2), thereby blocking the down-regulation of antitumor T-cell functions (U.S. Patent No. 8,008,449; Wang et al., 2014 Cancer Immunol Res. 2(9):846-56).

Ipilimumab (YERVOY^{®}) is a fully human, IgG1 monoclonal antibody that blocks the binding of CTLA-4 to its B7 ligands, thereby stimulating T cell activation and improving overall survival (OS) in patients with advanced melanoma (Hodi et al. (2010) N Engl J Med 363:711-23). Concurrent therapy with nivolumab and ipilimumab in a Phase 1 clinical trial produced rapid and deep tumor regression in a substantial proportion of patients with advanced melanoma, and was significantly more effective than either antibody alone (Wolchok et al. (2013) N Engl J Med 369(2):122-33; WO 2013/173223).

Urothelial carcinoma (UC) includes carcinomas of the bladder, ureters, and renal pelvis, with the vast majority of urothelial carcinomas presenting in the form of bladder cancer. Bladder cancer accounts for approximately 5% of all new cancers in the US, and it is estimated that in 2016, about 76,960 new cases of bladder cancer will be diagnosed, and about 16,390 people will die due to this disease ("Key statistics for bladder cancer," cancer.org, May 23, 2016). Although considered a chemosensitive disease, most patients with advanced or metastatic urothelial carcinoma relapse after cisplatin based first line treatment (*see, e.g.,* Oing et al., J. Urology 195(2):254-63 (2016)). Though many patients initially respond to standard of care treatment, most cases progress on average at about 8 months. On relapse, there are few treatment options. None of the commonly used drugs, *i.e.,* paclitaxel, carboplatin and/or gemcitabine, are approved by the FDA (Food and Drug Administration) for second line systemic treatment, and most patients upon relapse receive palliative care (*id*.). Accordingly, there remains a need for effective therapies for the treatment of UC, and, in particular, second line therapies for patients that relapse following initial treatment.

### SUMMARY OF THE INVENTION

The present disclosure relates to a method for treating a subject afflicted with a urothelial carcinoma (UC) or cancer derived therefrom comprising administering to the subject an antibody or an antigen-binding portion thereof that binds specifically to a Programmed Death-1 (PD-1) receptor and inhibits PD-1 activity ("anti-PD-1 antibody"). In some embodiments, the method further comprises administering to the subject an antibody or an antigen-binding portion thereof that binds specifically to Cytotoxic T-Lymphocyte Antigen-4 (CTLA-4) and inhibits CTLA-4 activity ("anti-CTLA-4 antibody").

Other aspects of the present disclosure relate to a method for treating a subject afflicted with a UC, or a cancer derived therefrom, comprising administering to the subject a combination of: (a) an anti-PD-1 antibody and (b) an anti-CTLA-4 antibody.

In some embodiments, the UC comprises a bladder cancer. In other embodiments, the UC comprises a carcinoma of the ureter. In other embodiments, the UC comprises a carcinoma of the renal pelvis. In some embodiments, the UC comprises a transitional cell carcinoma. In some embodiments, the UC comprises a squamous cell carcinoma. In some embodiments, the UC comprises an adenocarcinoma. In some embodiments, the UC is a recurrent UC. In some embodiments, the UC is locally advanced. In certain embodiments, the UC is metastatic.

In some embodiments, the subject received at least one, at least two, at least three, at least four, or at least five previous lines of therapy to treat the UC. In some embodiments, the previous line of therapy comprises a chemotherapy. In some embodiments, the chemotherapy comprises a platinum-based therapy. In some embodiments, the platinum-based therapy comprises a platinum-based antineoplastic selected from the group consisting of cisplatin, carboplatin, oxaliplatin, nedaplatin, triplatin tetranitrate, phenanthriplatin, picoplatin, satraplatin, and any combination thereof. In certain embodiments, the platinum-based therapy comprises cisplatin. In one particular embodiment, the platinum-based therapy comprises carboplatin.

In some embodiments, the anti-PD-1 antibody cross-competes with nivolumab for binding to human PD-1. In some embodiments, the anti-PD-1 antibody binds to the same epitope as nivolumab. In some embodiments, the anti-PD-1 antibody is a chimeric, humanized or human monoclonal antibody or a portion thereof. In other embodiments, the anti-PD-1 antibody comprises a heavy chain constant region which is of a human IgG1 or IgG4 isotype. In certain embodiments, the anti-PD-1 antibody is nivolumab. In one embodiment, the anti-PD-1 antibody is pembrolizumab.

In some embodiments, the anti-CTLA-4 antibody is a chimeric, humanized or human monoclonal antibody or a portion thereof. In some embodiments, the anti-CTLA-4 antibody comprises a heavy chain constant region which is of a human IgG1 isotype. In certain embodiments, the anti-CTLA-4 antibody is ipilimumab. In other embodiments, the anti-CTLA-4 antibody is tremelimumab. In some embodiments, the anti-CTLA-4 antibody cross-competes with ipilimumab for binding to human CTLA-4.

In some embodiments, the anti-PD-1 antibody is administered at a dose ranging from at least about 0.1 mg/kg to at least about 10.0 mg/kg body weight once about every 1, 2, 3, or 4 weeks. In some embodiments, the anti-PD-1 antibody is administered at a dose of about 1 mg/kg or about 3 mg/kg body weight. In some embodiments, the anti-PD-1 antibody is administered at a flat dose. In some embodiments, the anti-PD-1 antibody is administered at a flat dose of at least about 200 mg, at least about 220 mg, at least about 240 mg, at least about 260 mg, at least about 280 mg, at least about 300 mg, at least about 320 mg, at least about 340 mg, at least about 360 mg, at least about 380 mg, at least about 400 mg, at least about 420 mg, at least about 440 mg, at least about 460 mg, at least about 480 mg, at least about 500 mg or at least about 550 mg. In some embodiments, the anti-PD-1 antibody is administered at a flat dose about once every 1, 2, 3 or 4 weeks. In some embodiments, the anti-PD-1 antibody is administered once about every 2 weeks. In some embodiments, the anti-PD-1 antibody is administered once about every 3 weeks. In some embodiments, the anti-PD-1 antibody is administered for as long as clinical benefit is observed or until unmanageable toxicity or disease progression occurs.

In some embodiments, the anti-CTLA-4 antibody is administered at a dose ranging from at least about 0.1 mg/kg to at least about 10.0 mg/kg body weight once about every 1, 2, 3, or 4 weeks. In some embodiments, the anti-CTLA-4 antibody is administered at a dose of about 1 mg/kg or about 3 mg/kg body weight. In some embodiments, the anti-PD-1 antibody is administered at a flat dose. In some embodiments, the anti-CTLA-4 antibody is administered once about every 2 weeks. In some embodiments, the anti-CTLA-4 antibody is administered once about every 3 weeks.

In some embodiments, the anti-PD-1 antibody is administered at a dose of about 3 mg/kg body weight once about every 3 weeks and the anti-CTLA-4 antibody is administered at a dose of about 1 mg/kg body weight once about every 3 weeks. In some embodiments, the anti-PD-1 antibody is administered at a dose of about 1 mg/kg body weight once about every 3 weeks and the anti-CTLA-4 antibody is administered at a dose of about 3 mg/kg body weight once about every 3 weeks.

In certain embodiments, a subject treated with a disclosed method exhibits progression-free survival of at least about one month, at least about 2 months, at least about 3 months, at least about 4 months, at least about 5 months, at least about 6 months, at least about 7 months, at least about 8 months, at least about 9 months, at least about 10 months, at least about 11 months, at least about one year, at least about eighteen months, at least about two years, at least about three years, at least about four years, or at least about five years after the initial administration.

In some embodiments, the subject has a tumor that has ≥ 1% PD-L1 expression. In other embodiments, the subject has a tumor that has ≥ 5% PD-L1 expression. In certain embodiments, the anti-PD-1 antibody or the anti-PD-1 antibody and anti-CTLA-4 combination is administered for as long as clinical benefit is observed or until disease progression or unmanageable toxicity occurs. In one embodiment, the anti-PD-1 and/or anti-CTLA-4 antibodies are formulated for intravenous administration. In certain embodiments, the anti PD-1 antibody and the anti-CTLA-4 antibody are administered sequentially to the subject. In some embodiments, the anti-PD-1 and anti-CTLA-4 antibodies are administered within 30 minutes of each other. In one embodiment, the anti-PD-1 antibody is administered before the anti-CTLA-4 antibody. In another embodiment, the anti-CTLA-4 antibody is administered before the anti-PD-1 antibody. In some embodiments, the anti-PD-1 antibody and the anti-CTLA-4 antibody are administered concurrently in separate compositions. In certain embodiments, the anti-PD-1 antibody and the anti-CTLA-4 antibody are administered concurrently as a single composition.

In one embodiment, the anti-PD-1 antibody is administered at a subtherapeutic dose. In certain embodiments, the anti-CTLA-4 antibody is administered at a subtherapeutic dose. In some embodiments, the anti-PD-1 antibody and the anti-CTLA-4 antibody are each administered at a subtherapeutic dose.

The present disclosure further relates to a kit for treating a subject afflicted with a UC, or a cancer derived therefrom, the kit comprising: (a) an amount ranging from about 4 mg to about 500 mg of an anti-PD-1 antibody; and (b) instructions for using the anti-PD-1 antibody in any disclosed method.

The present disclosure further relates to a kit for treating a subject afflicted with a UC, or a cancer derived therefrom, the kit comprising: (a) an amount ranging from about 4 mg to about 500 mg of an anti-PD-1 antibody; (b) an amount ranging from about 4 mg to about 500 mg of an anti-CTLA-4 antibody; and (c) instructions for using the anti-PD-1 antibody and the anti-CTLA-4 antibody in any disclosed method.

Other features and advantages of the instant invention will be apparent from the following detailed description and examples which should not be construed as limiting. The contents of all cited references, including scientific articles, newspaper reports, GenBank entries, patents and patent applications cited throughout this application are expressly incorporated herein by reference.

### BRIEF DESCRIPTION OF THE DRAWINGS

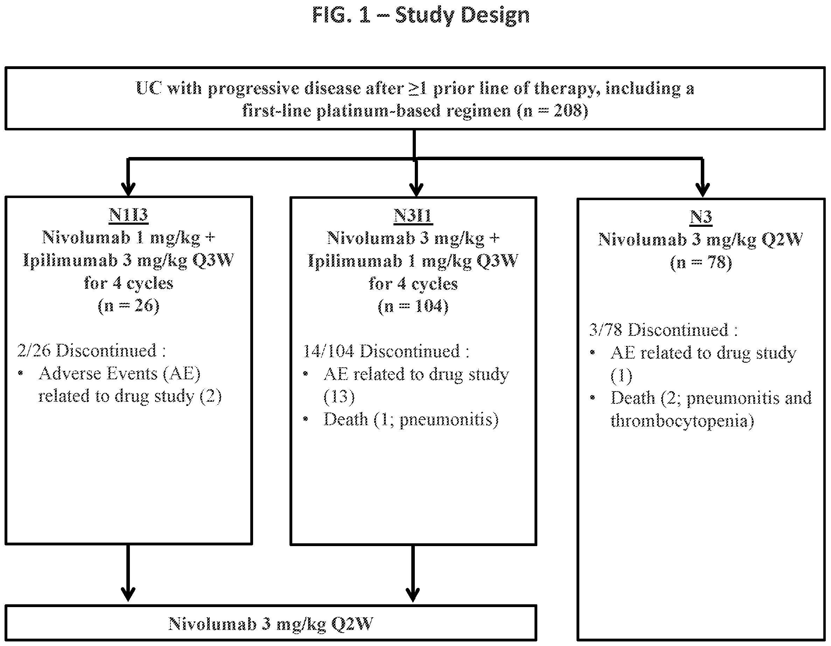

FIG. 1 shows a schematic representation of a study design for treatment of locally advanced or metastatic urothelial carcinoma (UC) previously treated with platinum-based therapy using an anti-PD-1 antibody or a combination of an anti-PD-1 antibody and an anti-CTLA-4 antibody.

### DETAILED DESCRIPTION OF THE INVENTION

The present invention relates to methods for treating a UC or a cancer derived therefrom in a patient comprising administering to the patient an anti-PD-1 antibody or a combination of an anti-PD-1 antibody and an anti-CTLA-4 antibody.

### Terms

In order that the present disclosure can be more readily understood, certain terms are first defined. As used in this application, except as otherwise expressly provided herein, each of the following terms shall have the meaning set forth below. Additional definitions are set forth throughout the application.

"Administering" refers to the physical introduction of a composition comprising a therapeutic agent to a subject, using any of the various methods and delivery systems known to those skilled in the art. Routes of administration for an antibody of the application (e.g., the anti-PD-1 antibody and/or anti-CTLA-4 antibody) include intravenous, intramuscular, subcutaneous, intraperitoneal, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion, as well as *in vivo* electroporation. In some embodiments, the combination is administered via a non-parenteral route, in some embodiments, orally. Other non-parenteral routes include a topical, epidermal or mucosal route of administration, for example, intranasally, vaginally, rectally, sublingually or topically. Administering can also be performed, for example, once, a plurality of times, and/or over one or more extended periods. In some embodiments, the anti-PD-1 antibody and the anti-CTLA-4 antibody can be administered concurrently or sequentially. In some embodiments, the anti-PD-1 antibody is administered before the anti-CTLA-4 antibody. In other embodiments, the anti-CTLA-4 antibody is administered before the anti-PD-1 antibody.

An "adverse event" (AE) as used herein is any unfavorable and generally unintended or undesirable sign (including an abnormal laboratory finding), symptom, or disease associated with the use of a medical treatment. For example, an adverse event can be associated with activation of the immune system or expansion of immune system cells (*e.g.,* T cells) in response to a treatment. A medical treatment can have one or more associated AEs and each AE can have the same or different level of severity. Reference to methods capable of "altering adverse events" means a treatment regime that decreases the incidence and/or severity of one or more AEs associated with the use of a different treatment regime.

An "antibody" (Ab) shall include, without limitation, a glycoprotein immunoglobulin which binds specifically to an antigen and comprises at least two heavy (H) chains and two light (L) chains interconnected by disulfide bonds, or an antigen-binding portion thereof. Each H chain comprises a heavy chain variable region (abbreviated herein as V*_{H}*) and a heavy chain constant region. The heavy chain constant region comprises three constant domains, C_{*H*1}, C_{*H*2} and C_{*H*3}. Each light chain comprises a light chain variable region (abbreviated herein as V*_{L}*) and a light chain constant region. The light chain constant region is comprises one constant domain, C*_{L}*. The V*_{H}* and V*_{L}* regions can be further subdivided into regions of hypervariability, termed complementarity determining regions (CDRs), interspersed with regions that are more conserved, termed framework regions (FR). Each V*_{H}* and V*_{L}* comprises three CDRs and four FRs, arranged from amino-terminus to carboxy-terminus in the following order: FR1, CDR1, FR2, CDR2, FR3, CDR3, and FR4. The variable regions of the heavy and light chains contain a binding domain that interacts with an antigen. The constant regions of the antibodies can mediate the binding of the immunoglobulin to host tissues or factors, including various cells of the immune system (*e.g.,* effector cells) and the first component (C1q) of the classical complement system.

An immunoglobulin can derive from any of the commonly known isotypes, including but not limited to IgA, secretory IgA, IgG and IgM. IgG subclasses are also well known to those in the art and include but are not limited to human IgG1, IgG2, IgG3 and IgG4. "Isotype" refers to the antibody class or subclass (*e.g.,* IgM or IgG1) that is encoded by the heavy chain constant region genes. The term "antibody" includes, by way of example, both naturally occurring and non-naturally occurring antibodies; monoclonal and polyclonal antibodies; chimeric and humanized antibodies; human or nonhuman antibodies; wholly synthetic antibodies; and single chain antibodies. A nonhuman antibody can be humanized by recombinant methods to reduce its immunogenicity in man. Where not expressly stated, and unless the context indicates otherwise, the term "antibody" also includes an antigen-binding fragment or an antigen-binding portion of any of the aforementioned immunoglobulins, and includes a monovalent and a divalent fragment or portion, and a single chain antibody.

An "isolated antibody" refers to an antibody that is substantially free of other antibodies having different antigenic specificities (*e.g.,* an isolated antibody that binds specifically to PD-1 is substantially free of antibodies that bind specifically to antigens other than PD-1). An isolated antibody that binds specifically to PD-1 can, however, have cross-reactivity to other antigens, such as PD-1 molecules from different species. Moreover, an isolated antibody can be substantially free of other cellular material and/or chemicals.

The term "monoclonal antibody" (mAb) refers to a non-naturally occurring preparation of antibody molecules of single molecular composition, *i.e.,* antibody molecules whose primary sequences are essentially identical, and which exhibits a single binding specificity and affinity for a particular epitope. A monoclonal antibody is an example of an isolated antibody. Monoclonal antibodies can be produced by hybridoma, recombinant, transgenic or other techniques known to those skilled in the art.

A "human" antibody (HuMAb) refers to an antibody having variable regions in which both the framework and CDR regions are derived from human germline immunoglobulin sequences. Furthermore, if the antibody contains a constant region, the constant region is also derived from human germline immunoglobulin sequences. The human antibodies of the disclosure can include amino acid residues not encoded by human germline immunoglobulin sequences (*e.g.,* mutations introduced by random or site-specific mutagenesis *in vitro* or by somatic mutation *in vivo*). However, the term "human antibody," as used herein, is not intended to include antibodies in which CDR sequences derived from the germline of another mammalian species, such as a mouse, have been grafted onto human framework sequences. The terms "human" antibodies and "fully human" antibodies and are used synonymously.

A "humanized antibody" refers to an antibody in which some, most or all of the amino acids outside the CDR domains of a non-human antibody are replaced with corresponding amino acids derived from human immunoglobulins. In one embodiment of a humanized form of an antibody, some, most or all of the amino acids outside the CDR domains have been replaced with amino acids from human immunoglobulins, whereas some, most or all amino acids within one or more CDR regions are unchanged. Small additions, deletions, insertions, substitutions or modifications of amino acids are permissible as long as they do not abrogate the ability of the antibody to bind to a particular antigen. A "humanized" antibody retains an antigenic specificity similar to that of the original antibody.

A "chimeric antibody" refers to an antibody in which the variable regions are derived from one species and the constant regions are derived from another species, such as an antibody in which the variable regions are derived from a mouse antibody and the constant regions are derived from a human antibody.

An "anti-antigen" antibody refers to an antibody that binds specifically to the antigen. For example, an anti-PD-1 antibody binds specifically to PD-1 and an anti-CTLA-4 antibody binds specifically to CTLA-4.

An "antigen-binding portion" of an antibody (also called an "antigen-binding fragment") refers to one or more fragments of an antibody that retain the ability to bind specifically to the antigen bound by the whole antibody.

As used herein, a first antibody is said to "cross-compete" with a second antibody if both the first antibody and the second antibody are capable of binding the same antigen, and the binding of the first antibody to the antigen inhibits the binding of the second antibody to the antigen. In some embodiments, the first and second antibody bind the same epitope on the antigen. In some embodiments, the first antibody and second antibody bind epitopes which overlap with each other or which are adjacent to each other. In other embodiments, the first antibody and second antibody bind different epitopes which are distal to each other on the antigen. In some embodiments, the first antibody physically blocks the second antibody from binding the epitope by masking the epitope. In other embodiments, the binding of the antigen by the first antibody causes a conformational change in the three-dimensional structure of the antigen that inhibits the ability of the second antibody to bind the antigen. Cross-competition can be determined using any techniques known in the art for measuring antibody-antigen interactions, including, but not limited to, BIACORE^{®} analysis, ELISA assays, immunohistochemistry, and flow cytometry. The ability of a test antibody to inhibit the binding of a reference antibody demonstrates that the test antibody can compete with the reference antibody.

A "cancer" refers to various diseases characterized by the uncontrolled growth of abnormal cells in the body. A "cancer" or "cancer tissue" can include a tumor. Unregulated cell division and growth results in the formation of cancer (*e.g.,* malignant tumors) that invade neighboring tissues or lymph nodes (referred to herein as "locally advanced") and can also metastasize to distant parts of the body through the lymphatic system or bloodstream (referred to herein as "metastatic"). Locally advanced cancer can be "derived from" the original, pre-invading cancer or tumor. Following metastasis, the cancer (e.g., distal tumor) can also be "derived from" the original, pre-metastasis cancer or tumor. For example, a "cancer derived from" a UC includes a cancer or tumor that is the result of a locally advanced or metastasized urothelial carcinoma.

"Cytotoxic T-Lymphocyte Antigen-4 (CTLA-4) refers to an immunoinhibitory receptor belonging to the CD28 family. CTLA-4 is expressed exclusively on T cells *in vivo,* and binds to two ligands, CD80 and CD86 (also called B7-1 and B7-2, respectively). The term "CTLA-4" as used herein includes human CTLA-4 (hCTLA-4), variants, isoforms, and species homologs of hCTLA-4, and analogs having at least one common epitope with hCTLA-4. The complete hCTLA-4 sequence can be found under GenBank Accession No. AAB59385.

The term "immunotherapy" refers to the treatment of a subject afflicted with, or at risk of contracting or suffering a recurrence of, a disease by a method comprising inducing, enhancing, suppressing or otherwise modifying an immune response.

"Treatment," "treating," or "therapy" of a subject refers to any type of intervention or process performed on, or the administration of an active agent to, the subject with the objective of reversing, alleviating, ameliorating, inhibiting, slowing down or preventing the onset, progression, development, severity or recurrence of a symptom, complication or condition, or biochemical indicia associated with a disease. As used herein, "progression free survival" refers to the period of time wherein the disease (*e.g.,* UC or cancer derived therefrom) does not progress and the subject remains alive.

"PD-L1 positive" as used herein can be interchangeably used with "PD-L1 expression of at least about 1%." In one embodiment, the PD-L1 expression can be used by any methods known in the art. In another embodiment, the PD-L1 expression is measured by an automated IHC. PD-L1 positive tumor can thus have at least about 1%, at least about 2%, at least about 5%, at least about 10%, or at least about 20% of tumor cells expressing PD-L1 as measured by an automated IHC. In certain embodiments, "PD-L1 positive" means that there are at least 100 cells that express PD-L1 on the surface of the cells.

"Programmed Death-1 (PD-1)" refers to an immunoinhibitory receptor belonging to the CD28 family. PD-1 is expressed predominantly on previously activated T cells *in vivo,* and binds to two ligands, PD-L1 and PD-L2. The term "PD-1" as used herein includes human PD-1 (hPD-1), variants, isoforms, and species homologs of hPD-1, and analogs having at least one common epitope with hPD-1. The complete hPD-1 sequence can be found under GenBank Accession No. U64863.

"Programmed Death Ligand-1 (PD-L1)" is one of two cell surface glycoprotein ligands for PD-1 (the other being PD-L2) that downregulate T cell activation and cytokine secretion upon binding to PD-1. The term "PD-L1" as used herein includes human PD-L1 (hPD-L1), variants, isoforms, and species homologs of hPD-L1, and analogs having at least one common epitope with hPD-L1. The complete hPD-L1 sequence can be found under GenBank Accession No. Q9NZQ7.

A "subject" includes any human or nonhuman animal. The term "nonhuman animal" includes, but is not limited to, vertebrates such as nonhuman primates, sheep, dogs, and rodents such as mice, rats and guinea pigs. In some embodiments, the subject is a human. The terms, "subject" and "patient" are used interchangeably herein.

As used herein, a subject is said to be "afflicted with" a disease or disorder if the subject has the disease or disorder (e.g., a UC or cancer derived therefrom). In some embodiments, the subject has been diagnosed as having a UC or cancer derived therefrom. In some embodiments, the UC is a recurrent UC following one or more previous therapy. In some embodiments, the subject is receiving treatment for the UC or cancer derived therefrom. In certain embodiments, the subject has been diagnosed with a UC, but does not present with any signs or symptoms commonly associated with a UC. In some embodiments, the subject previously had a UC, which is no longer detectable following one or more previous therapy.

A "therapeutically effective amount" or "therapeutically effective dosage" of a drug or therapeutic agent is any amount of the drug that, when used alone or in combination with another therapeutic agent, protects a subject against the onset of a disease or promotes disease regression evidenced by a decrease in severity of disease symptoms, an increase in frequency and duration of disease symptom-free periods, or a prevention of impairment or disability due to the disease affliction. The ability of a therapeutic agent to promote disease regression can be evaluated using a variety of methods known to the skilled practitioner, such as in human subjects during clinical trials, in animal model systems predictive of efficacy in humans, or by assaying the activity of the agent in *in vitro* assays.

As used herein, "subtherapeutic dose" means a dose of a therapeutic compound (*e.g.,* an antibody) that is lower than the usual or typical dose of the therapeutic compound when administered alone for the treatment of a hyperproliferative disease (*e.g.,* cancer).

By way of example, an "anti-cancer agent" promotes cancer regression in a subject or prevents further tumor growth. In certain embodiments, a therapeutically effective amount of the drug promotes cancer regression to the point of eliminating the cancer. "Promoting cancer regression" means that administering an effective amount of the drug, alone or in combination with an anti-neoplastic agent, results in a reduction in tumor growth or size, necrosis of the tumor, a decrease in severity of at least one disease symptom, an increase in frequency and duration of disease symptom-free periods, or a prevention of impairment or disability due to the disease affliction. In addition, the terms "effective" and "effectiveness" with regard to a treatment includes both pharmacological effectiveness and physiological safety. Pharmacological effectiveness refers to the ability of the drug to promote cancer regression in the patient. Physiological safety refers to the level of toxicity, or other adverse physiological effects at the cellular, organ and/or organism level (adverse effects) resulting from administration of the drug.

By way of example for the treatment of tumors, a therapeutically effective amount of an anti-cancer agent can inhibit cell growth or tumor growth by at least about 20%, by at least about 40%, by at least about 60%, or by at least about 80% relative to untreated subjects. In other embodiments of the disclosure, tumor regression can be observed and continue for a period of at least about 20 days, at least about 40 days, or at least about 60 days. Notwithstanding these ultimate measurements of therapeutic effectiveness, evaluation of immunotherapeutic drugs must also make allowance for "immune-related" response patterns.

An "immune-related" response pattern refers to a clinical response pattern often observed in cancer patients treated with immunotherapeutic agents that produce antitumor effects by inducing cancer-specific immune responses or by modifying native immune processes. This response pattern is characterized by a beneficial therapeutic effect that follows an initial increase in tumor burden or the appearance of new lesions, which in the evaluation of traditional chemotherapeutic agents would be classified as disease progression and would be synonymous with drug failure. Accordingly, proper evaluation of immunotherapeutic agents can require long-term monitoring of the effects of these agents on the target disease.

A therapeutically effective amount of a drug includes a "prophylactically effective amount," which is any amount of the drug that, when administered alone or in combination with an anti-neoplastic agent to a subject at risk of developing a cancer (*e.g.,* a subject having a pre-malignant condition) or of suffering a recurrence of cancer, inhibits the development or recurrence of the cancer. In certain embodiments, the prophylactically effective amount prevents the development or recurrence of the cancer entirely. "Inhibiting" the development or recurrence of a cancer means either lessening the likelihood of the cancer's development or recurrence, or preventing the development or recurrence of the cancer entirely.

A "recurrent" UC refers to a UC that has progressed after one or more previous lines of therapy. In some embodiments, the UC progression occurred after a partial response or a complete response to one or more previous lines of therapy.

A "previous line of therapy," as used herein, refers to any therapy administered to the subject for the treatment of a UC or a cancer derived therefrom that occurred prior to or at the same time as the recurrence of the UC. In certain embodiments, the previous line of therapy comprises a chemotherapy. In some embodiments, the chemotherapy comprises a platinum-based therapy. In certain embodiments, the platinum based therapy comprises a platinum-based antineoplastic agent selected from the group consisting of cisplatin, carboplatin, oxaliplatin, nedaplatin, triplatin tetranitrate, phenanthriplatin, picoplatin, satraplatin, and any combination thereof. In one particular embodiment, the platinum-based therapy comprises cisplatin (*e.g.,* cisplatin alone; in combination with methotrexate, vinblastine, and/or doxorubicin ("MVAC"); or in combination with paclitaxel and/or gemcitabine ("PGC")). In other embodiments, the chemotherapy comprises paclitaxel, docetaxel, gemcitabine, or any combination thereof. In some embodiments, previous line of therapy comprises a radiotherapy.

The use of the alternative (*e.g.,* "or") should be understood to mean either one, both, or any combination thereof of the alternatives. As used herein, the indefinite articles "a" or "an" should be understood to refer to "one or more" of any recited or enumerated component.

The terms "about" or "comprising essentially of" refer to a value or composition that is within an acceptable error range for the particular value or composition as determined by one of ordinary skill in the art, which will depend in part on how the value or composition is measured or determined, i.e., the limitations of the measurement system. For example, "about" or "comprising essentially of" can mean within 1 or more than 1 standard deviation per the practice in the art. Alternatively, "about" or "comprising essentially of" can mean a range of up to 10% or 20% (*i.e.,* ± 10% or ± 20%). For example, about 3mg can include any number between 2.7 mg and 3.3 mg (for 10%) or between 2.4 mg and 3.6 mg (for 20%). Furthermore, particularly with respect to biological systems or processes, the terms can mean up to an order of magnitude or up to 5-fold of a value. When particular values or compositions are provided in the application and claims, unless otherwise stated, the meaning of "about" or "comprising essentially of" should be assumed to be within an acceptable error range for that particular value or composition.

The terms "once about every week," "once about every two weeks," or any other similar dosing interval terms as used herein mean approximate numbers. "Once about every week" can include every seven days ± one day, *i.e.*, every six days to every eight days. "Once about every two weeks" can include every fourteen days ± three days, *i.e.,* every eleven days to every seventeen days. Similar approximations apply, for example, to once about every three weeks, once about every four weeks, once about every five weeks, once about every six weeks and once about every twelve weeks. In some embodiments, a dosing interval of once about every six weeks or once about every twelve weeks means that the first dose can be administered any day in the first week, and then the next dose can be administered any day in the sixth or twelfth week, respectively. In other embodiments, a dosing interval of once about every six weeks or once about every twelve weeks means that the first dose is administered on a particular day of the first week (e.g., Monday) and then the next dose is administered on the same day of the sixth or twelfth weeks (i.e., Monday), respectively.

As described herein, any concentration range, percentage range, ratio range or integer range is to be understood to include the value of any integer within the recited range and, when appropriate, fractions thereof (such as one tenth and one hundredth of an integer), unless otherwise indicated.

Various aspects of the invention are described in further detail in the following subsections.

### Methods of the Invention

This disclosure provides a method of treating a subject afflicted with a UC, or a cancer derived therefrom, which method comprises administering to the subject an antibody or an antigen-binding portion thereof that binds specifically to a Programmed Death-1 (PD-1) receptor and inhibits PD-1 activity ("anti-PD-1 antibody"). This disclosure further provides a method of treating a subject afflicted with a UC, or a cancer derived therefrom, which method comprises administering to the subject a combination of (a) an anti-PD-1 antibody; and (b) an antibody or an antigen-binding portion thereof that binds specifically to Cytotoxic T-Lymphocyte Antigen-4 (CTLA-4) and inhibits CTLA-4 activity ("anti-CTLA-4 antibody"). In some embodiments, the subject is a human patient.

In certain embodiments, the subject is a chemotherapy-naive patient (*e.g.,* a patient who has not previously received any chemotherapy). In other embodiments, the subject has received another cancer therapy (*e.g.,* a chemotherapy), but is resistant or refractory to such another cancer therapy. In one particular embodiment, the UC is a recurrent UC. In some embodiments, the subject received at least one, at least two, at least three, at least four, or at least five previous lines of therapy to treat the tumor. In one embodiment, the subject received one previous line of therapy to treat the tumor. In another embodiment, the subject received two previous lines of therapy to treat the tumor. In another embodiment, the subject received three previous lines of therapy to treat the tumor. In another embodiment, the subject received four previous lines of therapy to treat the tumor. In another embodiment, the subject received five previous lines of therapy to treat the tumor. In another embodiment, the subject received more than five previous lines of therapy to treat the tumor.

In certain embodiments, the previous line of therapy comprised a chemotherapy. In some embodiments, the chemotherapy comprises a platinum-based therapy. In certain embodiments, the platinum based therapy comprises a platinum-based antineoplastic selected from the group consisting of cisplatin, carboplatin, oxaliplatin, nedaplatin, triplatin tetranitrate, phenanthriplatin, picoplatin, satraplatin, and any combination thereof. In one particular embodiment, the platinum-based therapy comprises cisplatin (*e.g.,* cisplatin alone; in combination with methotrexate, vinblastine, and/or doxorubicin ("MVAC"); or in combination with paclitaxel and/or gemcitabine ("PGC")). In other embodiments, the chemotherapy comprises paclitaxel, docetaxel, gemcitabine, or any combination thereof. In some embodiments, the subject has received a previous radiotherapy.

In certain specific embodiments, the subject has cancer cells expressing mutated forms of the *EGFR* or *KRAS* gene. In certain embodiments, the subject has cancer cells that are PD-L1 positive. In certain embodiments, the subject has cancer cells that are PD-L1 negative. In some embodiments, the subject never smoked. In certain embodiments, the subject formerly smoked. In one embodiments, the subject currently smokes. In some embodiments, the subject never ingested or abused one or more analgesic. In certain embodiments, the subject previously abused an analgesic. In one embodiments, the subject abuses one or more analgesic. I some embodiments, the analgesic comprises phenacetin. In other embodiments, the analgesic does not comprise phenacetin. In certain embodiments, abuse of an analgesic is characterized by consumption of more than 100 mg, more than 1.0 g, more than 10 g, more than 100 g, more than 1kg, more than 2 kg, more than 3 kg, more than 4 kg, more than 5 kg, or more than 10 kg. In one embodiment, abuse of an analgesic is characterized by consumption of more than 5 kg over the course of the patient's life.

In certain embodiments, the UC comprises a bladder carcinoma. In other embodiments, the UC comprises a carcinoma of the ureters. In still other embodiments, the UC comprises a carcinoma of the renal pelvis. In certain embodiments, the UC comprises a carcinoma of any one or more of the bladder, the ureters, and the renal pelvis.

In some embodiments, the UC comprises a transitional cell carcinoma. Transitional cell carcinomas arise from the urothelial cells lining the inside of the bladder, the ureters, and the renal pelvis.

In some embodiments, the UC comprises a squamous cell carcinoma. A squamous cell carcinoma, *e.g.,* of the bladder, arises from the bladder uroepithelium with pure squamous phenotype.

In some embodiments, the UC comprises an adenocarcinoma. An adenocarcinoma, *e.g.,* of the bladder, is defined as a tumor composed entirely of malignant glandular epithelium.

In certain embodiments, the UC or cancer derived therefrom comprises a bladder carcinoma, a carcinoma of the ureters, a carcinoma of the renal pelvis, a transitional cell carcinoma, a squamous cell cancer, an adenocarcinoma, or any combination thereof.

In some embodiments, the present methods comprise administering an effective amount of an anti-PD-1 antibody or administering an effective amount of an anti-PD-L1 antibody and an effective amount of an anti-CTLA-4 antibody. An effective amount of an anti-PD-1 antibody and/or an anti-CTLA-4 antibody can be a flat dose or a weight based dose.

In embodiments, the invention includes a method of treating a cancer or a subject afflicted with cancer comprising administering an anti-PD-1 antagonist in combination with an anti-CTLA-4 antibody to treat cancer. An "anti-PD-1 antagonist" as referred herein includes any molecule that inhibits interaction between PD-1 (receptor) and PD-L1 (ligand) such that the signal pathway of PD-1/PD-L1 is blocked. In other embodiments, an anti-PD-1 antagonist is a PD-1-Fc fusion protein. In certain embodiments, an anti-PD-1 antagonist includes an anti-PD-1 fusion protein, an antisense molecule, a small molecule, a ribozyme, or a nanobody that inhibits or prevents interaction between PD-1 and PD-L1.

In certain embodiments, the therapy of the present invention (*e.g.,* administration of an anti-PD-1 antibody or administration of an anti-PD-1 antibody and an anti-CTLA-4 antibody) effectively increases the duration of survival of the subject. In some embodiments, the anti-PD-1 antibody or the anti-PD-1 antibody and anti-CTLA-4 antibody combination therapy of the present invention increases the progression-free survival of the subject. In certain embodiments, the anti-PD-1 antibody or the anti-PD-1 antibody and anti-CTLA-4 antibody combination therapy of the present invention increases the progression-free survival of the subject in comparison to standard-of-care therapies. In some embodiments, the anti-PD-1 antibody and anti-CTLA-4 antibody combination therapy of the present invention increases the progression-free survival of the subject in comparison to an anti-PD-1 antibody alone. In some embodiments, the anti-PD-1 antibody and anti-CTLA-4 antibody combination therapy of the present invention increases the progression-free survival of the subject in comparison to other anti-PD-1 antibody combinations.

In some embodiments, after the administration of an anti-PD-1 antibody or administration of an anti-PD-1 antibody and an anti-CTLA-4 antibody, the subject having a UC, or a cancer derived therefrom, can exhibit an overall survival of at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months at least about 15 months, at least about 16 months, at least about 17 months, at least about 18 months, at least about 19 months, at least about 20 months, at least about 21 months, at least about 22 months, at least about 23 months, at least about 2 years, at least about 3 years, at least about 4 years, or at least about 5 years after the administration.

In other embodiments, after the administration of a therapy disclosed herein (*e.g.,* an anti-PD-1 antibody therapy or an anti-PD-1 antibody and an anti-CTLA-4 antibody therapy), the duration of survival or the overall survival of the subject is increased by at least about 1 month, at least about 2 months, at least about 3 months, at least about 4 months, at least about 6 months, at least about 1 year, at least about 2 years, at least about 3 years, at least about 4 years, at least about 5 years, at least about 10 years, or at least about 15 years when compared to another subject treated with only a standard-of-care therapy (*e.g.,* a platinum-based chemotherapy) or a different dosing schedule of the therapy. For example, the duration of survival or the overall survival of the subject treated with an anti-PD-1 antibody disclosed herein is increased by at least about 5%, at least about 10%, at least about 15%, at least about 20%, at least about 25%, at least about 30%, at least about 40%, at least about 50%, at least about 75%, at least about 100%, at least about 200%, at least about 300%, or at least about 500% when compared to another subject treated with only a standard-of-care therapy (*e.g.,* a platinum-based chemotherapy) or a different dosing schedule of the anti-PD-1 antibody therapy.

In other embodiments, after the administration of a combination therapy comprising an anti-PD-1 antibody and an anti-CTLA-4 antibody, the duration of survival or the overall survival of the subject is increased by at least about 1 month, at least about 2 months, at least about 3 months, at least about 4 months, at least about 6 months, at least about 1 year, at least about 2 years, at least about 3 years, at least about 4 years, at least about 5 years, at least about 10 years, or at least about 15 years when compared to another subject treated with only a standard-of-care therapy (*e.g.,* a platinum-based chemotherapy), an anti-PD1 antibody alone, or a different dosing schedule of the combination therapy. For example, the duration of survival or the overall survival of the subject treated with an anti-PD-1 antibody and an anti-CTLA-4 antibody combination therapy disclosed herein is increased by at least about 5%, at least about 10%, at least about 15%, at least about 20%, at least about 25%, at least about 30%, at least about 40%, at least about 50% at least about 75%, at least about 100%, at least about 200%, at least about 300%, or at least about 500% when compared to another subject treated with only a standard-of-care therapy (*e.g.,* a platinum-based chemotherapy), an anti-PD-1 antibody alone, or a different dosing schedule of the combination therapy.

In certain embodiments, the therapy of the present invention effectively increases the duration of progression free survival of the subject. In some embodiments, the subject exhibits a progression-free survival of at least about one month, at least about 2 months, at least about 3 months, at least about 4 months, at least about 5 months, at least about 6 months, at least about 7 months, at least about 8 months, at least about 9 months, at least about 10 months, at least about 11 months, at least about one year, at least about eighteen months, at least about two years, at least about three years, at least about four years, at least about five years, at least about ten years, at least about fifteen years.

In some embodiments, the anti-PD-1 and anti-CTLA-4 antibodies are formulated for intravenous administration. In certain embodiments, the anti-PD-1 and anti-CTLA-4 antibodies are administered sequentially. In embodiments, the anti-PD-1 and anti-CTLA-4 antibodies are administered within 30 minutes of each other. In one embodiment, the anti-PD-1 antibody is administered before the anti-CTLA-4 antibody. In another embodiment, the anti-CTLA-4 antibody is administered before the anti-PD-1 antibody. In another embodiment, the anti-PD-1 antibody and the anti-CTLA-4 antibody are administered concurrently in separate compositions. In a further embodiment, the anti-PD-1 antibody and the anti-CTLA-4 antibody are admixed as a single composition for concurrent administration.

In some embodiments, the anti-PD-1 antibody and anti-CTLA-4 antibody are administered in a fixed dose.

In some embodiments, the PD-L1 status of a tumor in a subject is measured prior to administering any composition or utilizing any method disclosed herein. In one embodiment, the PD-L1 expression level of a tumor is at least about 1%, at least about 2%, at least about 3%, at least about 4%, at least about 5%, at least about 6%, at least about 7%, at least about 8%, at least about 9%, at least about 10%, at least about 11%, at least about 12%, at least about 13%, at least about 14%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, or at least about 95%. In another embodiment, the PD-L1 status of a tumor is at least about 1%. In other embodiments, the PD-L1 status of the subject is at least about 5%. In other embodiments, the PD-L1 status of a tumor is at least about 10%. In other embodiments, the PD-L1 status of a tumor is at least about 20%. In other embodiments, the PD-L1 status of a tumor is at least about 30%. In other embodiments, the PD-L1 status of a tumor is at least about 40%. In other embodiments, the PD-L1 status of a tumor is at least about 50%. In other embodiments, the PD-L1 status of a tumor is at least about 60%. In other embodiments, the PD-L1 status of a tumor is at least about 70%. In other embodiments, the PD-L1 status of a tumor is at least about 80%. In other embodiments, the PD-L1 status of a tumor is at least about 90%.

In some embodiments, the median progression-free survival of a subject with a tumor that has ≥ 1% PD-L1 expression is at least about 1 week, at least about 2 weeks, at least about 3 weeks, at least about 4 weeks, at least about 1 month, at least about 2 months, at least about 3 months, at least about 4 months, at least about 5 months, at least about 6 months, at least about 7 months, at least about 8 months, at least about 9 months, at least about 10 months, at least about 11 months, or at least about 1 year longer than the median progression-free survival of a subject with a tumor with a < 1% PD-L1 expression. In some embodiments, the progression-free survival of a subject with a tumor that has ≥ 1% PD-L1 expression is at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 1 year, at least about eighteen months, at least about 2 years, at least about 3 years, at least about 4 years, or at least about 5 years.

In order to assess the PD-L1 expression, in one embodiment, a test tissue sample can be obtained from the patient who is in need of the therapy. In another embodiment, the assessment of PD-L1 expression can be achieved without obtaining a test tissue sample. In some embodiments, selecting a suitable patient includes (i) optionally providing a test tissue sample obtained from a patient with cancer of the tissue, the test tissue sample comprising tumor cells and/or tumor-infiltrating inflammatory cells; and (ii) assessing the proportion of cells in the test tissue sample that express PD-L1 on the surface of the cells based on an assessment that the proportion of cells in the test tissue sample that express PD-L1 on the cell surface is higher than a predetermined threshold level.

In any of the methods comprising the measurement of PD-L1 expression in a test tissue sample, however, it should be understood that the step comprising the provision of a test tissue sample obtained from a patient is an optional step. It should also be understood that in certain embodiments the "measuring" or "assessing" step to identify, or determine the number or proportion of, cells in the test tissue sample that express PD-L1 on the cell surface is performed by a transformative method of assaying for PD-L1 expression, for example by performing a reverse transcriptase-polymerase chain reaction (RT-PCR) assay or an IHC assay. In certain other embodiments, no transformative step is involved and PD-L1 expression is assessed by, for example, reviewing a report of test results from a laboratory. In certain embodiments, the steps of the methods up to, and including, assessing PD-L1 expression provides an intermediate result that may be provided to a physician or other healthcare provider for use in selecting a suitable candidate for the anti-PD-1 antibody or anti-PD-L1 antibody therapy. In certain embodiments, the steps that provide the intermediate result is performed by a medical practitioner or someone acting under the direction of a medical practitioner. In other embodiments, these steps are performed by an independent laboratory or by an independent person such as a laboratory technician.

In certain embodiments of any of the present methods, the proportion of cells that express PD-L1 is assessed by performing an assay to determine the presence of PD-L1 RNA. In further embodiments, the presence of PD-L1 RNA is determined by RT-PCR, *in situ* hybridization or RNase protection. In other embodiments, the proportion of cells that express PD-L1 is assessed by performing an assay to determine the presence of PD-L1 polypeptide. In further embodiments, the presence of PD-L1 polypeptide is determined by immunohistochemistry (IHC), enzyme-linked immunosorbent assay (ELISA), *in vivo* imaging, or flow cytometry. In some embodiments, PD-L1 expression is assayed by IHC. In other embodiments of all of these methods, cell surface expression of PD-L1 is assayed using, *e.g.,* IHC or *in vivo* imaging.

Imaging techniques have provided important tools in cancer research and treatment. Recent developments in molecular imaging systems, including positron emission tomography (PET), single-photon emission computed tomography (SPECT), fluorescence reflectance imaging (FRI), fluorescence-mediated tomography (FMT), bioluminescence imaging (BLI), laser-scanning confocal microscopy (LSCM) and multiphoton microscopy (MPM), will likely herald even greater use of these techniques in cancer research. Some of these molecular imaging systems allow clinicians to not only see where a tumor is located in the body, but also to visualize the expression and activity of specific molecules, cells, and biological processes that influence tumor behavior and/or responsiveness to therapeutic drugs (Condeelis and Weissleder, "In vivo imaging in cancer," Cold Spring Harb. Perspect. Biol. 2(12):a003848 (2010)). Antibody specificity, coupled with the sensitivity and resolution of PET, makes immunoPET imaging particularly attractive for monitoring and assaying expression of antigens in tissue samples (McCabe and Wu, "Positive progress in immunoPET-not just a coincidence," Cancer Biother. Radiopharm. 25(3):253-61 (2010); Olafsen et al., "ImmunoPET imaging of B-cell lymphoma using 124I-anti-CD20 scFv dimers (diabodies)," Protein Eng. Des. Sel. 23(4):243-9 (2010)). In certain embodiments of any of the present methods, PD-L1 expression is assayed by immunoPET imaging. In certain embodiments of any of the present methods, the proportion of cells in a test tissue sample that express PD-L1 is assessed by performing an assay to determine the presence of PD-L1 polypeptide on the surface of cells in the test tissue sample. In certain embodiments, the test tissue sample is a FFPE tissue sample. In other embodiments, the presence of PD-L1 polypeptide is determined by IHC assay. In further embodiments, the IHC assay is performed using an automated process. In some embodiments, the IHC assay is performed using an anti-PD-L1 monoclonal antibody to bind to the PD-L1 polypeptide.

In one embodiment of the present methods, an automated IHC method is used to assay the expression of PD-L1 on the surface of cells in FFPE tissue specimens. This disclosure provides methods for detecting the presence of human PD-L1 antigen in a test tissue sample, or quantifying the level of human PD-L1 antigen or the proportion of cells in the sample that express the antigen, which methods comprise contacting the test sample, and a negative control sample, with a monoclonal antibody that specifically binds to human PD- L1, under conditions that allow for formation of a complex between the antibody or portion thereof and human PD-L1. In certain embodiments, the test and control tissue samples are FFPE samples. The formation of a complex is then detected, wherein a difference in complex formation between the test sample and the negative control sample is indicative of the presence of human PD-L1 antigen in the sample. Various methods are used to quantify PD-L1 expression.

In a particular embodiment, the automated IHC method comprises: (a) deparaffinizing and rehydrating mounted tissue sections in an autostainer; (b) retrieving antigen using a decloaking chamber and pH 6 buffer, heated to 110°C for 10 min; (c) setting up reagents on an autostainer; and (d) running the autostainer to include steps of neutralizing endogenous peroxidase in the tissue specimen; blocking non-specific protein-binding sites on the slides; incubating the slides with primary antibody; incubating with a postprimary blocking agent; incubating with NovoLink Polymer; adding a chromogen substrate and developing; and counterstaining with hematoxylin.

For assessing PD-L1 expression in tumor tissue samples, a pathologist examines the number of membrane PD-L1⁺ tumor cells in each field under a microscope and mentally estimates the percentage of cells that are positive, then averages them to come to the final percentage. The different staining intensities are defined as 0/negative, 1+/weak, 2+/moderate, and 3+/strong. Typically, percentage values are first assigned to the 0 and 3+ buckets, and then the intermediate 1+ and 2+ intensities are considered. For highly heterogeneous tissues, the specimen is divided into zones, and each zone is scored separately and then combined into a single set of percentage values. The percentages of negative and positive cells for the different staining intensities are determined from each area and a median value is given to each zone. A final percentage value is given to the tissue for each staining intensity category: negative, 1+, 2+, and 3+. The sum of all staining intensities needs to be 100%. In one embodiment, the threshold number of cells that needs to be PD-L1 positive is at least about 100, at least about 125, at least about 150, at least about 175, or at least about 200 cells. In certain embodiments, the threshold number or cells that needs to be PD-L1 positive is at least about 100 cells.

Staining is also assessed in tumor-infiltrating inflammatory cells such as macrophages and lymphocytes. In most cases macrophages serve as an internal positive control since staining is observed in a large proportion of macrophages. While not required to stain with 3+ intensity, an absence of staining of macrophages should be taken into account to rule out any technical failure. Macrophages and lymphocytes are assessed for plasma membrane staining and only recorded for all samples as being positive or negative for each cell category. Staining is also characterized according to an outside/inside tumor immune cell designation. "Inside" means the immune cell is within the tumor tissue and/or on the boundaries of the tumor region without being physically intercalated among the tumor cells. "Outside" means that there is no physical association with the tumor, the immune cells being found in the periphery associated with connective or any associated adjacent tissue.

In certain embodiments of these scoring methods, the samples are scored by two pathologists operating independently, and the scores are subsequently consolidated. In certain other embodiments, the identification of positive and negative cells is scored using appropriate software.

A histoscore is used as a more quantitative measure of the IHC data. The histoscore is calculated as follows: Histoscore = [(% tumor x 1 (low intensity)) + (% tumor x 2 (medium intensity)) + (% tumor x 3 (high intensity)]

To determine the histoscore, the pathologist estimates the percentage of stained cells in each intensity category within a specimen. Because expression of most biomarkers is heterogeneous the histoscore is a truer representation of the overall expression. The final histoscore range is 0 (no expression) to 300 (maximum expression).

An alternative means of quantifying PD-L1 expression in a test tissue sample IHC is to determine the adjusted inflammation score (AIS) score defined as the density of inflammation multiplied by the percent PD-L1 expression by tumor-infiltrating inflammatory cells (Taube et al., "Colocalization of inflammatory response with B7-h1 expression in human melanocytic lesions supports an adaptive resistance mechanism of immune escape," Sci. Transl. Med. 4(727):127ra37 (2012)).

The present methods can treat a UC, or a cancer derived therefrom, of any stage. There are at least six stages used for UC (*see* Edge et al. (eds), AJCC Cancer Staging Manual, 7th Edition, New York, Springer, 2010, which is incorporated by reference herein in its entirety): Stage 0a (Ta, N0, and M0), Stage 0is (carcinoma in situ; Tis, N0, and M0), Stage I (T1, N0, and M0), Stage II (T2a, N0, M0; or T2b, N0, and M0), Stage III (T3a, N0, and M0; T3b, N0, and M0; or T4a, N0, and M0), and Stage IV (T1-T3, N2, and M0; T3, N1, and M0; or T4, N0-N1, and M0), Stage IIIB (Any T, N3, M0; or T4, N2, and M0), and Stage IV (T4b, N0, and M0; any T, N1-N3, and M0; or any T, any N, and M1).

In one embodiment, the present methods treat a Stage I UC. In Stage I, the tumor has, *e.g.,* invaded the subepithelial convective tissue (lamina propria).

In another embodiment, the methods of the present invention treat a Stage II UC. Stage II UC can be characterized, *e.g.,* by either tumor invasion of the superficial muscularis propria (inner half) or invasion of the deep muscularis propria (outer half).

In other embodiments, any methods of the present invention treat Stage III UC. Stage III can be characterized, *e.g.,* by (1) microscopic invasion of the perivesical tissue, (2) macroscopic invasion of the perivesical tissue, or (3) invasion of the prostatic stroma, seminal vesicles, uterus, vagina, pelvic wall, abdominal wall, or any combination thereof.

In some embodiments, the methods of the invention treat a Stage IV UC. Stage IV UC can be characterized, *e.g.,* by (1) invasion of the pelvic wall and/or the abdominal wall; (2) any local metastasis combined with metastasis to one or more lymph node; or (3) any local metastasis combined with or without metastasis to one or more lymph node and distant metastasis.

### Anti-PD-1 Antibodies

Anti-PD-1 antibodies suitable for use in the disclosed methods are antibodies that bind to PD-1 with high specificity and affinity and inhibit PD-1 activity (*e.g.,* block the binding of PD-1 to PD-L1 and inhibit the immunosuppressive effect of the PD-1 signaling pathway). In any of the therapeutic methods disclosed herein, an anti-PD-1 or anti-PD-L1 "antibody" includes an antigen-binding portion that binds to the PD-1 or PD-L1 receptor, respectively, and exhibits the functional properties similar to those of whole antibodies in inhibiting ligand binding and upregulating the immune system. In certain embodiments, the anti-PD-1 antibody cross-competes with nivolumab for binding to human PD-1. In other embodiments, the anti-PD-L1 antibody competes for binding with BMS-936559, MPDL3280A, MEDI4736 or MSB0010718C for binding to human PD-L1.

In other embodiments, the anti-PD-1 antibody, or anti-PD-L1 antibody is a chimeric, humanized or human monoclonal antibody or a portion thereof. In certain embodiments for treating a human subject, the antibody is a humanized antibody In other embodiments for treating a human subject, the antibody is a human antibody antibodies of an IgG1, IgG2, IgG3 or IgG4 isotype can be used.

In certain embodiments, the anti-PD-1 antibody, or anti-PD-L1 antibody comprises a heavy chain constant region which is of a human IgG1 or IgG4 isotype. In certain other embodiments, the sequence of the IgG4 heavy chain constant region of the anti-PD-1 antibody, or anti-PD-L1 antibody contains an S228P mutation which replaces a serine residue in the hinge region with the proline residue normally found at the corresponding position in IgG1 isotype antibodies. This mutation, which is present in nivolumab, prevents Fab arm exchange with endogenous IgG4 antibodies, while retaining the low affinity for activating Fc receptors associated with wild-type IgG4 antibodies (Wang et al. In vitro characterization of the anti-PD-1 antibody nivolumab, BMS-936558, and in vivo toxicology in non-human primates, Cancer Imm Res, 2(9):846-56 (2014)). In yet other embodiments, the antibody comprises a light chain constant region which is a human kappa or lambda constant region. In other embodiments, the anti-PD-1 antibody, or anti-PD-L1 antibody, or antigen-binding portions thereof is a monoclonal antibody or an antigen-binding portion thereof.

Any anti-PD-1 antibody that is known in the art can be used in the presently described methods. In particular, various human monoclonal antibodies that bind specifically to PD-1 with high affinity have been disclosed in U.S. Patent No. 8,008,449. Other anti-PD-1 monoclonal antibodies have been described in, for example, U.S. Patent Nos. 6,808,710, 7,488,802, 8,168,757 and 8,354,509, and PCT Publication No. WO 2012/145493. Each of the anti-PD-1 human monoclonal antibodies disclosed in U.S. Patent No. 8,008,449 has been demonstrated to exhibit one or more of the following characteristics: (a) binds to human PD-1 with a K_{D} of 1 x 10⁻⁷ M or less, as determined by surface plasmon resonance using a Biacore biosensor system; (b) does not substantially bind to human CD28, CTLA-4 or ICOS; (c) increases T-cell proliferation in a Mixed Lymphocyte Reaction (MLR) assay; (d) increases interferon-γ production in an MLR assay; (e) increases IL-2 secretion in an MLR assay; (f) binds to human PD-1 and cynomolgus monkey PD-1; (g) inhibits the binding of PD-L1 and/or PD-L2 to PD-1; (h) stimulates antigen-specific memory responses; (i) stimulates antibody responses; and (j) inhibits tumor cell growth *in vivo.* Anti-PD-1 antibodies usable in the present invention include monoclonal antibodies that bind specifically to human PD-1 and exhibit at least one, in some embodiments, at least five, of the preceding characteristics. In some embodiments, the anti-PD-1 antibody is nivolumab. In one embodiment, the anti-PD-1 antibody is pembrolizumab.

Other anti-PD-1 monoclonal antibodies have been described in, for example, U.S. Patent Nos. 6,808,710, 7,488,802, 8,168,757 and 8,354,509, US Publication No. 2016/0272708, and PCT Publication Nos. WO 2012/145493, WO 2008/156712, WO 2015/112900, WO 2012/145493, WO 2015/112800, WO 2014/206107, WO 2015/35606, WO 2015/085847, WO 2014/179664, WO 2017/020291, WO 2017/020858, WO 2016/197367, WO 2017/024515, WO 2017/025051, WO 2017/123557, WO 2016/106159, WO 2014/194302, WO 2017/040790, WO 2017/133540, WO 2017/132827, WO 2017/024465, WO 2017/025016, WO 2017/106061, each of which is incorporated by reference in its entirety.

In some embodiments, the anti-PD-1 antibody is selected from the group consisting of nivolumab (also known as "OPDIVO^{®}"; formerly designated 5C4, BMS-936558, MDX-1106, or ONO-4538), pembrolizumab (Merck, also known as "KEYTRUDA^{®}", lambrolizumab, and MK-3475. *See* WO2008156712A1), PDR001 (Novartis; *see* WO 2015/112900), MEDI-0680 (AstraZeneca; AMP-514; *see* WO 2012/145493), REGN-2810 (Regeneron; *see* WO 2015/112800), JS001 (TAIZHOU JUNSHI PHARMA; *see* Si-Yang Liu et al., J. Hematol. Oncol. 10:136 (2017)), BGB-A317 (Beigene; *see* WO 2015/35606 and US 2015/0079109), INCSHR1210 (SHR-1210; Jiangsu Hengrui Medicine; *see* WO 2015/085847; Si-Yang Liu et al., J. Hematol. Oncol. 10:136 (2017)), TSR-042 (ANB011; Tesaro Biopharmaceutical; *see* WO2014/179664), GLS-010 (WBP3055; Wuxi/Harbin Gloria Pharmaceuticals; *see* Si-Yang Liu et al., J. Hematol. Oncol. 10:136 (2017)), AM-0001 (Armo), STI-1110 (Sorrento Therapeutics; *see* WO 2014/194302), AGEN2034 (Agenus; *see* WO 2017/040790), and MGD013 (Macrogenics).

In one embodiment, the anti-PD-1 antibody is nivolumab. Nivolumab is a fully human IgG4 (S228P) PD-1 immune checkpoint inhibitor antibody that selectively prevents interaction with PD-1 ligands (PD-L1 and PD-L2), thereby blocking the down-regulation of antitumor T-cell functions (U.S. Patent No. 8,008,449; Wang et al., Cancer Imm Res, 2(9):846-56 (2014)).

In another embodiment, the anti-PD-1 antibody is pembrolizumab. Pembrolizumab is a humanized monoclonal IgG4 antibody directed against human cell surface receptor PD-1 (programmed death-1 or programmed cell death-1). Pembrolizumab is described, for example, in U.S. Patent Nos. 8,354,509 and 8,900,587; *see also* www.cancer.gov/drugdictionary?cdrid=695789 (last accessed: December 14, 2014). Pembrolizumab has been approved by the FDA for the treatment of relapsed or refractory melanoma.

Anti-PD-1 antibodies usable in the disclosed methods also include isolated antibodies that bind specifically to human PD-1 and cross-compete for binding to human PD-1 with any anti-PD-1 antibody disclosed herein, e.g., nivolumab (*see, e.g.,* U.S. Patent No. 8,008,449 and 8,779,105; WO 2013/173223). In some embodiments, the anti-PD-1 antibody binds the same epitope as any of the anti-PD-1 antibodies described herein, e.g., nivolumab. The ability of antibodies to cross-compete for binding to an antigen indicates that these monoclonal antibodies bind to the same epitope region of the antigen and sterically hinder the binding of other cross-competing antibodies to that particular epitope region. These cross-competing antibodies are expected to have functional properties very similar those of the reference antibody, *e.g.,* nivolumab, by virtue of their binding to the same epitope region of PD-1. Cross-competing antibodies can be readily identified based on their ability to cross-compete with nivolumab in standard PD-1 binding assays such as Biacore analysis, ELISA assays or flow cytometry (*see, e.g.,* WO 2013/173223).

In certain embodiments, the antibodies that cross-compete for binding to human PD-1 with, or bind to the same epitope region of human PD-1 antibody, nivolumab are monoclonal antibodies. For administration to human subjects, these cross-competing antibodies are chimeric antibodies, engineered antibodies, or humanized or human antibodies. Such chimeric, engineered, humanized, or human monoclonal antibodies can be prepared and isolated by methods well known in the art.

Anti-PD-1 antibodies usable in the methods of the disclosed invention also include antigen-binding portions of the above antibodies. It has been amply demonstrated that the antigen-binding function of an antibody can be performed by fragments of a full-length antibody.

Anti-PD-1 antibodies suitable for use in the disclosed methods or compositions are antibodies that bind to PD-1 with high specificity and affinity, block the binding of PD-L1 and or PD-L2, and inhibit the immunosuppressive effect of the PD-1 signaling pathway. In any of the compositions or methods disclosed herein, an anti-PD-1 "antibody" includes an antigen-binding portion or fragment that binds to the PD-1 receptor and exhibits the functional properties similar to those of whole antibodies in inhibiting ligand binding and up-regulating the immune system. In certain embodiments, the anti-PD-1 antibody cross-competes with nivolumab for binding to human PD-1.

### Anti-PD-L1 Antibodies

Any anti-PD-L1 antibody can be used in the methods of the present disclosure. Examples of anti-PD-L1 antibodies useful in the methods of the present disclosure include the antibodies disclosed in US Patent No. 9,580,507. Each of the anti-PD-L1 human monoclonal antibodies disclosed in U.S. Patent No. 9,580,507 have been demonstrated to exhibit one or more of the following characteristics: (a) binds to human PD-L1 with a K_{D} of 1 × 10⁻⁷ M or less, as determined by surface plasmon resonance using a Biacore biosensor system; (b) increases T-cell proliferation in a Mixed Lymphocyte Reaction (MLR) assay; (c) increases interferon-γ production in an MLR assay; (d) increases IL-2 secretion in an MLR assay; (e) stimulates antibody responses; and (f) reverses the effect of T regulatory cells on T cell effector cells and/or dendritic cells. Anti-PD-L1 antibodies usable in the present invention include monoclonal antibodies that bind specifically to human PD-L1 and exhibit at least one, in some embodiments, at least five, of the preceding characteristics.

In certain embodiments, the anti-PD-L1 antibody is selected from the group consisting of BMS-936559 (formerly 12A4 or MDX-1105; *see, e.g.,* U.S. Patent No. 7,943,743 and WO 2013/173223), MPDL3280A (also known as RG7446, atezolizumab, and TECENTRIQ^{®}; US 8,217,149; *see, also,* Herbst et al. (2013) J Clin Oncol 31(suppl):3000), durvalumab (IMFINZI^{™}; MEDI-4736; AstraZeneca; *see* WO 2011/066389), avelumab (Pfizer; MSB-0010718C; BAVENCIO^{®}; *see* WO 2013/079174), STI-1014 (Sorrento; *see* WO2013/181634), CX-072 (Cytomx; *see* WO2016/149201), KN035 (3D Med/Alphamab; *see* Zhang et al., Cell Discov. 7:3 (March 2017), LY3300054 (Eli Lilly Co.; *see, e.g,* WO 2017/034916), and CK-301 (Checkpoint Therapeutics; *see* Gorelik et al., AACR:Abstract 4606 (Apr 2016)).

In certain embodiments, the PD-L1 antibody is atezolizumab (TECENTRIQ^{®}). Atexolizumab is a fully humanized IgG1 monoclonal anti-PD-L1 antibody.

In certain embodiments, the PD-L1 antibody is durvalumab (IMFINZI^{™}). Durvalumab is a human IgG1 kappa monoclonal anti-PD-L1 antibody.

In certain embodiments, the PD-L1 antibody is avelumab (BAVENCIO^{®}). Avelumab is a human IgG1 lambda monoclonal anti-PD-L1 antibody.

In other embodiments, the anti-PD-L1 monoclonal antibody is selected from the group consisting of 28-8, 28-1, 28-12, 29-8, 5H1, and any combination thereof.

Anti-PD-L1 antibodies usable in the disclosed methods also include isolated antibodies that bind specifically to human PD-L1 and cross-compete for binding to human PD-L1 with any anti-PD-L1 antibody disclosed herein, *e.g.,* atezolizumab and/or avelumab. In some embodiments, the anti-PD-L1 antibody binds the same epitope as any of the anti-PD-L1 antibodies described herein, *e.g.,* atezolizumab and/or avelumab. The ability of antibodies to cross-compete for binding to an antigen indicates that these antibodies bind to the same epitope region of the antigen and sterically hinder the binding of other cross-competing antibodies to that particular epitope region. These cross-competing antibodies are expected to have functional properties very similar those of the reference antibody, *e.g.,* atezolizumab and/or avelumab, by virtue of their binding to the same epitope region of PD-L1. Cross-competing antibodies can be readily identified based on their ability to cross-compete with atezolizumab and/or avelumab in standard PD-L1 binding assays such as Biacore analysis, ELISA assays or flow cytometry (*see, e.g.,* WO 2013/173223).

In certain embodiments, the antibodies that cross-compete for binding to human PD-L1 with, or bind to the same epitope region of human PD-L1 antibody as, atezolizumab and/or avelumab, are monoclonal antibodies. For administration to human subjects, these cross-competing antibodies are chimeric antibodies, engineered antibodies, or humanized or human antibodies. Such chimeric, engineered, humanized or human monoclonal antibodies can be prepared and isolated by methods well known in the art.

Anti-PD-L1 antibodies usable in the methods of the disclosed invention also include antigen-binding portions of the above antibodies. It has been amply demonstrated that the antigen-binding function of an antibody can be performed by fragments of a full-length antibody.

Anti-PD-L1 antibodies suitable for use in the disclosed methods or compositions are antibodies that bind to PD-L1 with high specificity and affinity, block the binding of PD-1, and inhibit the immunosuppressive effect of the PD-1 signaling pathway. In any of the compositions or methods disclosed herein, an anti-PD-L1 "antibody" includes an antigen-binding portion or fragment that binds to PD-L1 and exhibits the functional properties similar to those of whole antibodies in inhibiting receptor binding and up-regulating the immune system. In certain embodiments, the anti-PD-L1 antibody cross-competes with atezolizumab and/or avelumab for binding to human PD-L1.

### Combination Therapies with Anti-PD-1 or Anti-PD-L1 Antibodies

In certain embodiments, an anti-PD-1 antibody or anti-PD-L1 antibody is administered in combination with one or more other anti-cancer agents. In certain embodiments, the one or more anti-cancer agents have been administered to the subject prior to the administration of the anti-PD-1 or anti-PD-L1 antibodies or prior to the combination with the anti-PD-1 or anti-PD-L1 antibodies. In certain embodiments, the one or more anti-cancer agents were not effective in treating the cancer. In some embodiments, the other anti-cancer agent is any anti-cancer agent described herein or known in the art. In certain embodiments, the other anti-cancer agent is an anti-CTLA-4 antibody. In one embodiment, the other anti-cancer agent is a chemotherapy or a platinum-based doublet chemotherapy (PT-DC). In certain embodiments, the other anti-cancer agent is an EGFR-targeted tyrosine kinase inhibitor (TKI). In one embodiment, the other anti-cancer agent is an anti-VEGF antibody. In other embodiments, the anti-cancer agent is a platinum agent (*e.g.,* cisplatin, carboplatin, oxaliplatin, and satraplatin), a mitotic inhibitor (*e.g.,* paclitaxel, albumin-bound paclitaxel, docetaxel, taxotere, docecad, vinblastine, doxorubicin, and eribulin), a fluorinated Vinca alkaloid (*e.g.,* vinflunine and javlor), a PI3K/AKT/mTOR inhibitor (*e.g.,* sirolimus, temsirolimus, and everolimus), an epidermal growth factor receptor (EGFR) inhibitor (*e.g.,* gefitinib, cetuximab, erlotinib, and panitumumab), a HER2 inhibitor (*e.g.,* trastuzumab and lapatinib), a fibroblast growth factor receptor (FGFR) inhibitor (*e.g.,* dovitinib), a vascular endothelial growth factor (VEGF) inhibitor (*e.g.,* bevacizumab, aflibercept, ramucirumab, and sunitinib), a MET/hepatocyte growth factor 1 (HGF1) inhibitor (*e.g.,* cabozantinib), vinorelbine, vinblastine, etoposide, pemetrexed, gemcitabin, cabazitaxel, fluorouracil, topotecan, pazopanib, pyrazoloacridine, pralatrexat, piritrexim, trimetrexate, ixabepilone, irinotecan, ifosfamide, interleukin-2, irinotecan, arsenic trioxide, or any combination thereof. In one embodiment, the other anti-cancer agent is 5-flurouracil (5-FU). In certain embodiments, the other anti-cancer agent is any other anti-cancer agent known in the art. In some embodiments, two or more additional anti-cancer agents are administered in combination with the anti-PD-1 or anti-PD-L1 antibody. In some embodiments, the PD-1 or PD-L1 antibody is combined with surgical resection and/or radiation therapy.

### Anti-CTLA-4 antibodies

Any anti-CTLA-4 antibody that is known in the art can be used in the methods of the present disclosure. Anti-CTLA-4 antibodies of the instant invention bind to human CTLA-4 so as to disrupt the interaction of CTLA-4 with a human B7 receptor. Because the interaction of CTLA-4 with B7 transduces a signal leading to inactivation of T-cells bearing the CTLA-4 receptor, disruption of the interaction effectively induces, enhances or prolongs the activation of such T cells, thereby inducing, enhancing or prolonging an immune response.

Human monoclonal antibodies that bind specifically to CTLA-4 with high affinity have been disclosed in U.S. Patent Nos. 6,984,720 and 7,605,238. Other anti-CTLA-4 monoclonal antibodies have been described in, for example, U.S. Patent Nos. 5,977,318, 6,051,227, 6,682,736, and 7,034,121 and International Publication Nos. WO 2012/122444, WO 2007/113648, WO 2016/196237, and WO 2000/037504, each of which is incorporated by reference herein in its entirety. The anti- CTLA-4 human monoclonal antibodies disclosed in U.S. Patent No. Nos. 6,984,720 and 7,605,238 have been demonstrated to exhibit one or more of the following characteristics: (a) binds specifically to human CTLA-4 with a binding affinity reflected by an equilibrium association constant (K*ₐ*) of at least about 10⁷ M⁻¹, or about 10⁹ M⁻¹, or about 10¹⁰ M⁻¹ to 10¹¹ M⁻¹ or higher, as determined by Biacore analysis; (b) a kinetic association constant (k*ₐ*) of at least about 10³, about 10⁴, or about 10⁵ m⁻¹ s⁻¹; (c) a kinetic disassociation constant (k*_{d}*) of at least about 10³, about 10⁴, or about 10⁵ m⁻¹ s⁻¹; and (d) inhibits the binding of CTLA-4 to B7-1 (CD80) and B7-2 (CD86). Anti-CTLA-4 antibodies useful for the present invention include monoclonal antibodies that bind specifically to human CTLA-4 and exhibit at least one, at least two or, at least three of the preceding characteristics.

In certain embodiments, the CTLA-4 antibody is selected from the group consisting of ipilimumab (YERVOY^{®}; U.S. Patent No. 6,984,720), MK-1308 (Merck), AGEN-1884 (Agenus Inc.; WO 2016/196237), and tremelimumab (formerly ticilimumab, CP-675,206; AstraZeneca; *see, e.g.,* WO 2000/037504 and Ribas, Update Cancer Ther. 2(3): 133-39 (2007)). In particular embodiments, the anti-CTLA-4 antibody is ipilimumab.

In particular embodiments, the anti-CTLA-4 antibody is the human monoclonal antibody 10D1 (now known as ipilimumab and marketed as YERVOY^{®}) as disclosed in U.S. Patent No. 6,984,720. Ipilimumab is an anti-CTLA-4 antibody for use in the methods disclosed herein. Ipilimumab is a fully human, IgG1 monoclonal antibody that blocks the binding of CTLA-4 to its B7 ligands, thereby stimulating T cell activation and improving overall survival (OS) in patients with advanced melanoma.

In particular embodiments, the anti-CTLA-4 is tremelimumab (also known as CP-675,206). Tremelimumab is human IgG2 monoclonal anti-CTLA-4 antibody. Tremelimumab is described in WO/2012/122444, U.S. Publ. No. 2012/263677, or WO Publ. No. 2007/113648 A2.

In particular embodiments, the CTLA-4 antibody is MK-1308, which is an anti-CTLA-4 antibody under development by Merck.

In particular embodiments, the CTLA-4 antibody is AGEN-1884, which is a recombinant human monoclonal antibody to human CTLA-4, developed by Agenus Inc.

Anti-CTLA-4 antibodies usable in the disclosed methods also include isolated antibodies that bind specifically to human CTLA-4 and cross-compete for binding to human CTLA-4 with any anti-CTLA-4 antibody disclosed herein, *e.g.,* ipilimumab and/or tremelimumab. In some embodiments, the anti-CTLA-4 antibody binds the same epitope as any of the anti-CTLA-4 antibodies described herein, *e.g.,* ipilimumab and/or tremelimumab. The ability of antibodies to cross-compete for binding to an antigen indicates that these antibodies bind to the same epitope region of the antigen and sterically hinder the binding of other cross-competing antibodies to that particular epitope region. These cross-competing antibodies are expected to have functional properties very similar those of the reference antibody, *e.g.,* ipilimumab and/or tremelimumab, by virtue of their binding to the same epitope region of CTLA-4. Cross-competing antibodies can be readily identified based on their ability to cross-compete with ipilimumab and/or tremelimumab in standard CTLA-4 binding assays such as Biacore analysis, ELISA assays or flow cytometry (*see, e.g.,* WO 2013/173223).

In certain embodiments, the antibodies that cross-compete for binding to human CTLA-4 with, or bind to the same epitope region of human CTLA-4 as ipilimumab and/or tremelimumab, are monoclonal antibodies. For administration to human subjects, these cross-competing antibodies are chimeric antibodies, engineered antibodies, or humanized or human antibodies. Such chimeric, engineered, humanized or human monoclonal antibodies can be prepared and isolated by methods well known in the art.

Anti-CTLA-4 antibodies usable in the methods of the disclosed invention also include antigen-binding portions of the above antibodies. It has been amply demonstrated that the antigen-binding function of an antibody can be performed by fragments of a full-length antibody.

Anti-CTLA-4 antibodies suitable for use in the disclosed methods or compositions are antibodies that bind to CTLA-4 with high specificity and affinity, block the activity of CTLA-4, and disrupt the interaction of CTLA-4 with a human B7 receptor. In any of the compositions or methods disclosed herein, an anti-CTLA-4 "antibody" includes an antigen-binding portion or fragment that binds to CTLA-4 and exhibits the functional properties similar to those of whole antibodies in inhibiting the interaction of CTLA-4 with a human B7 receptor and up-regulating the immune system. In certain embodiments, the anti-CTLA-4 antibody cross-competes with ipilimumab and/or tremelimumab for binding to human CTLA-4.

### Combination of an anti-PD-1 antibody with an anti-CTLA-4 antibody for treating t7C

This disclosure provides combination therapy methods for treating a UC, or a cancer derived therefrom, wherein an anti-PD-1 antibody is combined with another anti-cancer agent that is an antibody or an antigen-binding portion thereof that binds specifically to CTLA-4 and inhibits CTLA-4 activity. The combination of the anti-PD-1 antibody, nivolumab, and the anti-CTLA-4 antibody, ipilimumab, has been demonstrated herein (*see* Example 1) to produce early, durable antitumor activity in a UC patients, particularly with specific dosing schedules. Accordingly, in certain embodiments, the anti-CTLA-4 antibody that is used in combination with the anti-PD-1 antibody is ipilimumab. In embodiments, the anti-CTLA-4 antibody is tremelimumab. In other embodiments, the anti-CTLA-4 antibody is an antibody or portion thereof that cross-competes with ipilimumab for binding to human CTLA-4. In certain other embodiments, the anti-CTLA-4 antibody is a chimeric, humanized or human monoclonal antibody or a portion thereof. In yet other embodiments, the anti-CTLA-4 antibody comprises a heavy chain constant region that is of a human IgG1 or IgG4 isotype. In some embodiments, the anti-CTLA-4 antibody comprises a heavy chain constant region that is of a human IgG1 isotype.

Because of durability of the clinical effect previously demonstrated with immunotherapy by inhibition of immune checkpoints (*see, e.g.,* WO 2013/173223), the combination treatment can include, in alternative embodiments, a finite number of doses, *e.g.,* about 1-10 doses, or can involve dosing at long intervals, *e.g.,* once about every 3-6 months or once about every 1-2 years or longer intervals.

In certain embodiments of the present methods, the anti-PD-1 antibody is nivolumab. In other embodiments, it is pembrolizumab. In yet other embodiments, the anti-CTLA-4 antibody is ipilimumab. In further embodiments, the anti-CTLA-4 antibody is tremelimumab. Typically, the anti-PD-1 and anti-CTLA-4 antibodies are formulated for intravenous administration. In certain embodiments, when the anti-PD-1 and anti-CTLA-4 antibodies are administered in combination, they are administered within 30 minutes of each other. Either antibody can be administered first, that is, in certain embodiments, the anti-PD-1 antibody is administered before the anti-CTLA-4 antibody, whereas in other embodiments, the anti-CTLA-4 antibody is administered before the anti-PD-1 antibody. Typically, each antibody is administered by intravenous infusion over a period of 60 minutes. In certain embodiments, the anti-PD-1 and anti-CTLA-4 antibodies are administered concurrently, either admixed as a single composition in a pharmaceutically acceptable formulation for concurrent administration, or concurrently as separate compositions with each antibody in a pharmaceutically acceptable formulation.

In certain embodiments, the anti-PD-1 antibody is administered at a subtherapeutic dose. In certain other embodiments, the anti-CTLA-4 antibody is administered at a subtherapeutic dose. In further embodiments, both the anti-PD-1 antibody and the anti-CTLA-4 antibody are each administered at a subtherapeutic dose.

### Standard-of-Care Therapies for t7C

Standard-of-care therapies for different types of cancer are well known by persons of skill in the art. For example, the National Comprehensive Cancer Network (NCCN), an alliance of 21 major cancer centers in the USA, publishes the NCCN Clinical Practice Guidelines in Oncology (NCCN GUIDELINES^{®}) that provide detailed up-to-date information on the standard-of-care treatments for a wide variety of cancers (*see* NCCN GUIDELINES^{®} (2014), available at: http://www.nccn.org/professionals/physician_gls/f_guidelines.asp, last accessed June 2, 2016).

Chemotherapy, Surgery, and radiation therapy (RT) are the three modalities commonly used to treat UC patients. The most commonly used initial chemotherapy regimens are methotrexate, vinblastine, doxorubicin, and cisplatin (MVAC) and gemcitabine plus cisplatin (GC).

Although many UC patients respond to initial treatments, including chemotherapy and/or surgery, patients with recurrent UC have fewer and less reliable options for treatment, with many patients receiving palliative care. Therefore, there is a particular unmet need among patients who have recurrent UC as there is a lack of an effective treatment after first line therapy.

### Pharmaceutical Compositions and Dosages

Therapeutic agents of the present invention can be constituted in a composition, *e.g.,* a pharmaceutical composition containing an antibody and a pharmaceutically acceptable carrier. As used herein, a "pharmaceutically acceptable carrier" includes any and all solvents, dispersion media, coatings, antibacterial and antifungal agents, isotonic and absorption delaying agents, and the like that are physiologically compatible. In one embodiment, the carrier for a composition containing an antibody is suitable for intravenous, intramuscular, subcutaneous, parenteral, spinal or epidermal administration (*e.g.,* by injection or infusion). A pharmaceutical composition of the invention can include one or more pharmaceutically acceptable salts, antioxidant, aqueous and non-aqueous carriers, and/or adjuvants such as preservatives, wetting agents, emulsifying agents and dispersing agents.

Dosage regimens are adjusted to provide the optimum desired response, *e.g.,* a maximal therapeutic response and/or minimal adverse effects. For administration of an anti-PD-1 antibody, as a monotherapy or in combination with another anti-cancer agent (*e.g.,* in combination with an anti-CTLA-4 antibody), the dosage can range from about 0.01 to about 20 mg/kg, about 0.1 to about 10 mg/kg, about 0.1 to about 5 mg/kg, about 1 to about 5 mg/kg, about 2 to about 5 mg/kg, about 7.5 to about 12.5 mg/kg, or about 0.1 to about 30 mg/kg of the subject's body weight. For example, dosages can be about 0.1, about 0.3, about 1, about 2, about 3, about 5 or about 10 mg/kg body weight, or about 0.3, about 1, about 2, about 3, or about 5 mg/kg body weight. The dosing schedule is typically designed to achieve exposures that result in sustained receptor occupancy (RO) based on typical pharmacokinetic properties of an antibody An exemplary treatment regime entails administration about once per week, once about every 2 weeks, once about every 3 weeks, once about every 4 weeks, once about a month, once about every 3-6 months or longer. In certain embodiments, an anti-PD-1 antibody such as nivolumab is administered to the subject once about every 2 weeks. In other embodiments, the antibody is administered once about every 3 weeks. The dosage and scheduling can change during a course of treatment. For example, a dosing schedule for anti-PD-1 monotherapy can comprise administering the antibody: (i) about every 2 weeks in about 6-week cycles; (ii) about every 4 weeks for about six dosages, then about every three months; (iii) about every 3 weeks; (iv) about 3- about 10 mg/kg once followed by about 1 mg/kg every about 2-3 weeks. Considering that an IgG4 antibody typically has a half-life of 2-3 weeks, a dosage regimen for an anti-PD-1 antibody of the invention comprises at least about 0.3- at least about 10 mg/kg body weight, at least about 1- at least about 5 mg/kg body weight, or at least about 1- at least about 3 mg/kg body weight via intravenous administration, with the antibody being given every about 14-21 days in up to about 6-week or about 12-week cycles until complete response or confirmed progressive disease. In certain embodiments, an anti-PD-1 monotherapy is administered at 3 mg/kg every 2 weeks until progressive disease or unacceptable toxicity. In some embodiments, the antibody treatment, or any combination treatment disclosed herein, is continued for at least about 1 month, at least about 3 months, at least about 6 months, at least about 9 months, at least about 1 year, at least about 18 months, at least about 24 months, at least about 3 years, at least about 5 years, or at least about 10 years.

When used in combinations with other cancer agents (*e.g.,* in combination with an anti-CTLA-4 antibody), the dosage of an anti-PD-1 antibody can be lowered compared to the monotherapy dose. Dosages of nivolumab that are lower than the typical 3 mg/kg, but not less than 0.001 mg/kg, are subtherapeutic dosages. The subtherapeutic doses of an anti-PD-1 antibody used in the methods herein are higher than 0.001 mg/kg and lower than 3mg/kg. In some embodiments, a subtherapeutic dose is about 0.001 mg/kg-about 1 mg/kg, about 0.01 mg/kg-about 1 mg/kg, about 0.1 mg/kg-about 1 mg/kg, or about 0.001 mg/kg-about 0.1 mg/kg body weight. In some embodiments, the subtherapeutic dose is at least about 0.001 mg/kg, at least about 0.005 mg/kg, at least about 0.01 mg/kg, at least about 0.05 mg/kg, at least about 0.1 mg/kg, at least about 0.5 mg/kg, or at least about 1.0 mg/kg body weight. Receptor-occupancy data from 15 subjects who received 0.3 mg/kg to 10 mg/kg dosing with nivolumab indicate that PD-1 occupancy appears to be dose-independent in this dose range. Across all doses, the mean occupancy rate was 85% (range, 70% to 97%), with a mean plateau occupancy of 72% (range, 59% to 81%). In some embodiments, 0.3 mg/kg dosing can allow for sufficient exposure to lead to maximal biologic activity. Receptor-occupancy data from 15 subjects who received 0.3 mg/kg to 10 mg/kg dosing with nivolumab indicate that PD-1 occupancy appears to be dose-independent in this dose range. Across all doses, the mean occupancy rate was 85% (range, 70% to 97%), with a mean plateau occupancy of 72% (range, 59% to 81%) (Brahmer et al. (2010) J Clin Oncol 28:3167-75). Thus, 0.3 mg/kg dosing can allow for sufficient exposure to lead to maximal biologic activity.

Although higher nivolumab monotherapy dosing up to about 10 mg/kg every two weeks has been achieved without reaching the maximum tolerated does (MTD), the significant toxicities reported in other trials of checkpoint inhibitors plus anti-angiogenic therapy (*see, e.g.,* Johnson et al. (2013) Cancer Immunol Res 1:373-77; Rini et al. (2011) Cancer 117:758-67) support the selection of a nivolumab dose lower than 10 mg/kg.

In certain embodiments, the dose of an anti-PD-1 antibody (or an anti-PD-L1 antibody) and/or the anti-CTLA-4 antibody is a fixed dose in a pharmaceutical composition. In other embodiments, the method of the present invention can be used with a flat dose (a dose given to a patient irrespective of the body weight of the patient). For example, a flat dose of a nivolumab can be about 240 mg. For example, a flat dose of pembrolizumab can be about 200 mg. In embodiments, the anti-PD-1 antibody is administered at a dose of about 240 mg. In embodiments, the anti-PD-1 antibody is administered at a dose of about 360 mg. In embodiments, the anti-PD-1 antibody is administered at a dose of about 480 mg. In one embodiment, 360 mg of the anti-PD-1 antibody or antigen binding fragment is administered once every 3 weeks. In another embodiment, 480 mg of the anti-PD-1 antibody or antigen binding fragment is administered once every 4 weeks.

Ipilimumab (YERVOY^{®}) is approved for the treatment of melanoma at 3 mg/kg given intravenously once every 3 weeks for 4 doses. Thus, in some embodiments, about 3 mg/kg is the highest dosage of ipilimumab used in combination with the anti-PD-1 antibody though, in certain embodiments, an anti-CTLA-4 antibody such as ipilimumab can be dosed within the range of about 0.3 to about 10 mg/kg, about 0.5 to about 10 mg/kg, about 0.5 to about 5 mg/kg, or about 1 to about 5 mg/kg. body weight about every two or three weeks when combined with nivolumab. In other embodiments, ipilimumab is administered on a different dosage schedule from nivolumab. In some embodiments, ipilimumab is administered about every week, about every two weeks, about every three weeks, about every 4 weeks, about every five weeks, about every six weeks, about every seven weeks, about every eight weeks, about every nine weeks, about every ten weeks, about every eleven weeks, about every twelve weeks or about every fifteen weeks. Dosages of ipilimumab that are lower than the typical 3 mg/kg every 3 weeks, but not less than 0.001 mg/kg, are subtherapeutic dosages. The subtherapeutic doses of an anti-CTLA-4 antibody used in the methods herein are higher than 0.001 mg/kg and lower than 3mg/kg. In some embodiments, a subtherapeutic dose is about 0.001 mg/kg-about 1 mg/kg, about 0.01 mg/kg-about 1 mg/kg, about 0.1 mg/kg-about 1 mg/kg, or about 0.001 mg/kg-about 0.1 mg/kg body weight. In some embodiments, the subtherapeutic dose is at least about 0.001 mg/kg, at least about 0.005 mg/kg, at least about 0.01 mg/kg, at least about 0.05 mg/kg, at least about 0.1 mg/kg, at least about 0.5 mg/kg, or at least about 1.0 mg/kg body weight. It has been shown that combination dosing of nivolumab at 3 mg/kg and ipilimumab at 3 mg/kg exceeded the MTD in a melanoma population, whereas a combination of nivolumab at 1 mg/kg plus ipilimumab at 3 mg/kg or nivolumab at 3 mg/kg plus ipilimumab at 1 mg/kg was found to be tolerable in melanoma patients (Wolchok et al., N Engl J Med 369(2): 122-33(2013)). Accordingly, although nivolumab is tolerated up to 10 mg/kg given intravenously every 2 weeks, in certain embodiments doses of the anti-PD-1 antibody do not exceed about 3 mg/kg when combined with ipilimumab. In certain embodiments, based on risk-benefit and PK-PD assessments, the dosage used comprises a combination of nivolumab at about 1 mg/kg plus ipilimumab at about 3 mg/kg, nivolumab at about 3 mg/kg plus ipilimumab at about 1 mg/kg, or nivolumab at about 3 mg/kg plus ipilimumab at about 3 mg/kg is used, each administered at a dosing frequency of once about every 2-4 weeks, in certain embodiments, once about every 2 weeks or once about every 3 weeks. In certain other embodiments, nivolumab is administered at a dosage of about 0.1, about 0.3, about 1, about 2, about 3 or about 5 mg/kg in combination with ipilimumab administered at a dosage of about 0.1, about 0.3, about 1, about 2, about 3 or about 5 mg/kg, once about every 2 weeks, once about every 3 weeks, or once about every 4 weeks.

In certain embodiments, the combination of an anti-PD-1 antibody and an anti-CTLA-4 antibody is administered intravenously to the subject in an induction phase about every 2 or 3 weeks for 1, 2, 3 or 4 administrations. In certain embodiments, the combination of nivolumab and ipilimumab is administered intravenously in the induction phase about every 2 weeks or about every 3 weeks for about 4 administrations. The induction phase is followed by a maintenance phase during which only the anti-PD-1 antibody is administered to the subject at a dosage of about 0.1, about 0.3, about 1, about 2, about 3, about 5 or about 10 mg/kg about every two or three weeks for as long as the treatment proves efficacious or until unmanageable toxicity or disease progression occurs. In certain embodiments, nivolumab is administered during the maintenance phase at a dose of about 3 mg/kg body about every 2 weeks.

In certain embodiments, the anti-PD-1 antibody and the anti-CTLA-4 antibody is formulated as a single composition, wherein the dose of the anti-PD1 antibody and the dose of the anti-CTLA-4 antibody are combined at a ratio of 1:50, 1:40, 1:30, 1:20, 1:10. 1:5, 1:3, 1:1, 3:1, 5:1, 10:1, 20:1, 30:1, 40:1, or 50:1. In other embodiments, the dose of the anti-CTLA-4 antibody is a fixed dose. In certain embodiments, the dose of the anti-CTLA-4 antibody is a flat dose, which is given to a patient irrespective of the body weight. In a specific embodiment, the flat dose of the anti-CTLA-4 antibody is about 80 mg.

For combination of nivolumab with other anti-cancer agents, these agents are administered at their approved dosages. Treatment is continued as long as clinical benefit is observed or until unacceptable toxicity or disease progression occurs. Nevertheless, in certain embodiments, the dosages of these anti-cancer agents administered are significantly lower than the approved dosage, *i.e.,* a subtherapeutic dosage, of the agent is administered in combination with the anti-PD-1 antibody. The anti-PD-1 antibody can be administered at the dosage that has been shown to produce the highest efficacy as monotherapy in clinical trials, *e.g.,* about 3 mg/kg of nivolumab administered once about every three weeks (Topalian et al., N Engl J Med 366:2443-54 (2012); Topalian et al., Curr Opin Immunol 24:207-12 (2012)), or at a significantly lower dose, *i.e.,* at a subtherapeutic dose. In certain embodiments, the anti-PD-1 antibody is administered at about 3 mg/kg once about every three weeks.

Dosage and frequency vary depending on the half-life of the antibody in the subject. In general, human antibodies show the longest half-life, followed by humanized antibodies, chimeric antibodies, and nonhuman antibodies. The dosage and frequency of administration can vary depending on whether the treatment is prophylactic or therapeutic. In prophylactic applications, a relatively low dosage is typically administered at relatively infrequent intervals over a long period of time. Some patients continue to receive treatment for the rest of their lives. In therapeutic applications, a relatively high dosage at relatively short intervals is sometimes required until progression of the disease is reduced or terminated, or until the patient shows partial or complete amelioration of symptoms of disease. Thereafter, the patient can be administered a prophylactic regime.

Actual dosage levels of the active ingredient or ingredients in the pharmaceutical compositions of the present invention can be varied so as to obtain an amount of the active ingredient which is effective to achieve the desired therapeutic response for a particular patient, composition, and mode of administration, without being unduly toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts. A composition of the present invention can be administered via one or more routes of administration using one or more of a variety of methods well known in the art. As will be appreciated by the skilled artisan, the route and/or mode of administration will vary depending upon the desired results.

Because anti-PD-1 and anti-PD-L1 target the same signaling pathway and have been shown in clinical trials to exhibit similar levels of efficacy in a variety of cancers, including renal cell carcinoma (*see* Brahmer et al. (2012) N Engl J Med 366:2455-65; Topalian et al. (2012a) N Engl J Med 366:2443-54; WO 2013/173223), an anti-PD-L1 antibody may be substituted for the anti-PD-1 antibody in any of the therapeutic methods disclosed herein. In certain embodiments, the anti-PD-L1 antibody is BMS-936559 (formerly 12A4 or MDX-1105) (*see, e.g.,* U.S. Patent No. 7,943,743; WO 2013/173223). In other embodiments, the anti-PD-L1 antibody is MPDL3280A (also known as RG7446) (*see, e.g.,* Herbst et al. (2013) J Clin Oncol 31(suppl):3000. Abstract; U.S. Patent No. 8,217,149) or MEDI4736 (Khleif (2013) In: Proceedings from the European Cancer Congress 2013; September 27-October 1, 2013; Amsterdam, The Netherlands. Abstract 802). In certain embodiments, the antibodies that cross-compete for binding to human PD-L1 with, or bind to the same epitope region of human anti-PD-L1 as the above-references PD-L1 antibodies are monoclonal antibodies. For administration to human subjects, these cross-competing antibodies can be chimeric antibodies, or can be humanized or human antibodies. Such chimeric, humanized or human monoclonal antibodies can be prepared and isolated by methods well known in the art.

### Kits

Also within the scope of the present invention are kits comprising an anti-PD-1 antibody and another anti-cancer agent for therapeutic uses. Kits typically include a label indicating the intended use of the contents of the kit and instructions for use. The term label includes any writing, or recorded material supplied on or with the kit, or which otherwise accompanies the kit. Accordingly, this disclosure provides a kit for treating a subject afflicted with a UC, or a cancer derived therefrom, the kit comprising: (a) an amount ranging from about 4 mg to about 500 mg of an anti-PD-1 antibody; and (b) instructions for using the anti-PD-1 antibody in any method disclosed herein. This disclosure further provides a kit for treating a subject afflicted with a UC, or a cancer derived therefrom, the kit comprising: (a) an amount ranging from about 4 mg to about 500 mg of an anti-PD-1 antibody; (b) an amount ranging from about 4 mg to about 500 mg of a CTLA-4 antibody; and (c) instructions for using the anti-PD-1 antibody and the CTLA-4 antibody in any method disclosed herein. In some embodiments, the kit contains the anti-PD-1 antibody and the CTLA-4 antibody as separation compositions. In some embodiments, the kit contains the anti-PD-1 antibody or an antigen-binding portion thereof and the CTLA-4 antibody as a single composition. In certain embodiments, the anti-PD-1, and the anti-CTLA-4 antibody can be co-packaged in unit dosage form. In certain embodiments for treating human patients, the kit comprises an anti-human PD-1 antibody disclosed herein, *e.g.,* nivolumab or pembrolizumab. In other embodiments, the kit comprises an anti-human CTLA-4 antibody disclosed herein, *e.g.,* ipilimumab or tremelimumab.

The present invention is further illustrated by the following example that should not be construed as further limiting. The contents of all references cited throughout this application are expressly incorporated herein by reference.

### EMBODIMENTS

E1. A method for treating a subject afflicted with a urothelial carcinoma (UC) or cancer derived therefrom comprising administering to the subject an antibody or an antigen-binding portion thereof that binds specifically to a Programmed Death-1 (PD-1) receptor and inhibits PD-1 activity ("anti-PD-1 antibody").

E2. The method of E1, further comprising administering to the subject an antibody or an antigen-binding portion thereof that binds specifically to Cytotoxic T-Lymphocyte Antigen-4 (CTLA-4) and inhibits CTLA-4 activity ("anti-CTLA-4 antibody").

E3. The method of E1 or E2, wherein the UC comprises a bladder cancer.

E4. The method of E1 or E2, wherein the UC comprises a carcinoma of the ureter.

E5. The method of E1 or E2, wherein the UC comprises a carcinoma of the renal pelvis.

E6. The method of any one of E1-E5, wherein the UC comprises a transitional cell carcinoma.

E7. The method of any one of E1-E5, wherein the UC comprises a squamous cell carcinoma.

E8. The method of any one of E1-E3, wherein the UC comprises an adenocarcinoma.

E9. The method of any one of E1-E8, wherein the UC is a recurrent UC.

E10. The method of any one of E1-E9, wherein the UC is locally advanced.

E11. The method of any one of E1-E9, wherein the UC is metastatic.

E12. The method of any one of E1-E11, wherein the subject received at least one, at least two, at least three, at least four, or at least five previous lines of therapy to treat the UC.

E13. The method of E12, wherein the previous line of therapy comprises a chemotherapy.

E14. The method of E13, wherein the chemotherapy comprises a platinum-based therapy.

E15. The method of E14, wherein the platinum-based therapy comprises a platinum-based antineoplastic selected from the group consisting of cisplatin, carboplatin, oxaliplatin, nedaplatin, triplatin tetranitrate, phenanthriplatin, picoplatin, satraplatin, and any combination thereof.

E16. The method of E14 or E15, wherein the platinum-based therapy comprises cisplatin.

E17. The method of E14 or E15, wherein the platinum-based therapy comprises carboplatin.

E18. The method of any one of E1-E17, wherein the anti-PD-1 antibody cross-competes with nivolumab for binding to human PD-1.

E19. The method of any one of E1-E18, wherein the anti-PD-1 antibody binds to the same epitope as nivolumab.

E20. The method of any one of E1-E19, wherein the anti-PD-1 antibody is a chimeric, humanized or human monoclonal antibody or a portion thereof.

E21. The method of any one of E1-E20, wherein the anti-PD-1 antibody comprises a heavy chain constant region which is of a human IgG1 or IgG4 isotype.

E22. The method of any one of E1-E21, wherein the anti-PD-1 antibody is nivolumab.

E23. The method of any one of E1-E21, wherein the anti-PD-1 antibody is pembrolizumab.

E24. The method of any one of E2-E23, wherein the anti-CTLA-4 antibody is a chimeric, humanized or human monoclonal antibody or a portion thereof.

E25. The method of any one of E2-E24, wherein the anti-CTLA-4 antibody comprises a heavy chain constant region which is of a human IgG1 isotype.

E26. The method of any one of E2-E25, wherein the anti-CTLA-4 antibody is ipilimumab.

E27. The method of any one of E2-E25, wherein the anti-CTLA-4 antibody is tremelimumab.

E28. The method of any one of E2-E27, wherein the anti-CTLA-4 antibody cross-competes with ipilimumab for binding to human CTLA-4.

E29. The method of any one of E1-E28, wherein the anti-PD-1 antibody is administered at a dose ranging from at least about 0.1 mg/kg to at least about 10.0 mg/kg body weight once about every 1, 2, 3, or 4 weeks.

E30. The method of any one of E1-E29, wherein the anti-PD-1 antibody is administered at a dose of about 1 mg/kg or about 3 mg/kg body weight.

E31. The method of any one of E1-E28, wherein the anti-PD-1 antibody or antigen-binding portion thereof is administered at a flat dose.

E32. The method of any one of E1-E28 and E31, wherein the anti-PD-1 antibody or antigen-binding portion thereof is administered at a flat dose of at least about 200, at least about 220, at least about 240, at least about 260, at least about 280, at least about 300, at least about 320, at least about 340, at least about 360, at least about 380, at least about 400, at least about 420, at least about 440, at least about 460, at least about 480, at least about 500 or at least about 550 mg.

E33. The method of any one of E1-E28, E31, and E32, wherein the anti-PD-1 antibody or antigen-binding portion thereof is administered at a flat dose about once every 1, 2, 3 or 4 weeks.

E34. The method of anyone of E1-E33, wherein the anti-PD-1 antibody is administered once about every 2 weeks.

E35. The method of anyone of E1-E33, wherein the anti-PD-1 antibody is administered once about every 3 weeks.

E36. The method of any one of E1-E35, wherein the anti-PD-1 antibody is administered for as long as clinical benefit is observed or until unmanageable toxicity or disease progression occurs.

E37. The method of any one of E2-E36, wherein the anti-CTLA-4 antibody is administered at a dose ranging from at least about 0.1 mg/kg to at least about 10.0 mg/kg body weight once about every 1, 2, 3, or 4 weeks.

E38. The method of any one of E2-E37, wherein the anti-CTLA-4 is administered at a dose of about 1 mg/kg or about 3 mg/kg body weight.

E39. The method of any one of E2-E38, wherein the anti-PD-1 antibody or antigen-binding portion thereof is administered at a flat dose.

E40. The method of anyone of E2-E39, wherein the anti-CTLA-4 antibody is administered once about every 2 weeks.

E41. The method of anyone of E2-E40, wherein the anti-CTLA-4 antibody is administered once about every 3 weeks.

E42. The method of any one of E2-E41, wherein the anti-PD-1 antibody is administered at a dose of about 3 mg/kg body weight once about every 3 weeks and the anti-CTLA-4 antibody or antigen-binding portion thereof is administered at a dose of about 1 mg/kg body weight once about every 3 weeks.

E43. The method of any one of E2-E41, wherein the anti-PD-1 antibody is administered at a dose of about 1 mg/kg body weight once about every 3 weeks and the anti-CTLA-4 antibody or antigen-binding portion thereof is administered at a dose of about 3 mg/kg body weight once about every 3 weeks.

E44. The method of any one of E1-E43, wherein the subject exhibits progression-free survival of at least about one month, at least about 2 months, at least about 3 months, at least about 4 months, at least about 5 months, at least about 6 months, at least about 7 months, at least about 8 months, at least about 9 months, at least about 10 months, at least about 11 months, at least about one year, at least about eighteen months, at least about two years, at least about three years, at least about four years, or at least about five years after the initial administration.

E45. The method of any one of E1-E44, wherein the subject has a tumor that has ≥ 1% PD-L1 expression.

E46. The method of any one of E1-E45, wherein the subject has a tumor that has ≥ 5% PD-L1 expression.

E47. The method of any one of E2-E46, wherein the combination is administered for as long as clinical benefit is observed or until disease progression or unmanageable toxicity occurs.

E48. The method of any one of E1-E47, wherein the anti-PD-1 antibody is formulated for intravenous administration.

E49. The method of any one of E2-E48, wherein the anti-CTLA-4 antibody is formulated for intravenous administration.

E50. The method of any one of E2-E49, wherein the anti PD-1 antibody and the anti-CTLA-4 antibody are administered sequentially to the subject.

E51. The method of any one of E2-E50, wherein the anti-PD-1 and anti-CTLA-4 antibodies are administered within 30 minutes of each other.

E52. The method of any one of E2-E51, wherein the anti-PD-1 antibody or antigen-binding portion thereof is administered before the anti-CTLA-4 antibody or antigen-binding portion thereof.

E53. The method of any one of E2-E51, wherein the anti-CTLA-4 antibody or antigen-binding portion thereof is administered before the anti-PD-1 antibody or antigen-binding portion thereof.

E54. The method of any one of E2-E49, wherein the anti-PD-1 antibody or antigen-binding portion thereof and the anti-CTLA-4 antibody or antigen-binding portion thereof are administered concurrently in separate compositions.

E55. The method of any one of E2-E49, wherein the anti-PD-1 antibody or antigen-binding portion thereof and the anti-CTLA-4 antibody or antigen-binding portion thereof are administered concurrently as a single composition.

E56. The method of any one of E1-E55, wherein the anti-PD-1 antibody or antigen-binding portion thereof is administered at a subtherapeutic dose.

E57. The method of any one of E2-E56, wherein the anti-CTLA-4 antibody or antigen-binding portion thereof is administered at a subtherapeutic dose.

E58. The method of any one of E2-E57, wherein the anti-PD-1 antibody or antigen-binding portion thereof and the anti-CTLA-4 antibody or antigen-binding portion thereof are each administered at a subtherapeutic dose.

E59. A kit for treating a subject afflicted with a UC or a cancer derived therefrom, the kit comprising:
(a) an amount ranging from about 4 mg to about 500 mg of an anti-PD-1 antibody or an antigen-binding portion thereof; and
(b) instructions for using the PD-1 antibody in the method of any of E1-58.

E60. A kit for treating a subject afflicted with a UC or a cancer derived therefrom, the kit comprising:
(a) an amount ranging from about 4 mg to about 500 mg of an anti-PD-1 antibody or an antigen-binding portion thereof;
(b) an amount ranging from about 4 mg to about 500 mg of a CTLA-4 antibody or an antigen-binding portion thereof; and
(c) instructions for using the PD-1 antibody and the CTLA-4 antibody in the method of any of E2-58.

### EXAMPLE 1

### Example 1

Reported herein are the first efficacy and safety results of combined nivolumab plus ipilimumab given at two different dosing schedules in an open-label, multicenter phase I/II study of patients with locally advanced or metastatic UC who progressed after prior platinum-based therapy.

### Materials and Methods

Patients with locally advanced or metastatic UC previously treated with platinum-based therapy were included in the study (FIG. 1). Patients were treated with (1) either of two combination schedules, 1 mg/kg nivolumab combined with 3 mg/kg ipilimumab ("N1I3") or 3 mg/kg nivolumab combined with 1 mg/kg ipilimumab ("N3I1") administered every 3 weeks for four cycles, each combination followed by nivolumab 3 mg/kg every 2 weeks; or (2) 3 mg/kg nivolumab monotherapy (N3) administered every 2 weeks. All patients were treated until disease progression or unacceptable toxicity. The primary endpoint was investigator-assessed objective response rate (ORR) by RECIST v1.1. Secondary endpoints included safety and duration of response (DoR).

### Results and Conclusions

The minimum follow-up was 3.9 months for the N1I3 (n = 26) group, 14.5 months for the N3I1 group (n = 104), and 13.8 months in N3 group (n = 78). The object response rate was 38.5%, 26.0%, and 25.6% in the N1I3, N3I1, and N3 groups, respectively (Table 1). The median duration of response (DoR) had not been reached in any treatment group at the time of this data collection. The frequency of drug-related grade 3-4 adverse events was 30.8% in N1I3, 31.7% in N3I1, and 23.1% in N3. Treatment-related adverse events led to discontinuation in 7.7% (N1I3), 13.5% (N3I1), and 3.8% (N3) of patients. One death was reported in the N3I1 group (pneumonitis), and two deaths were reported in the N3 group (pneumonitis and thrombocytopenia).

**Table 1: Tumor response**

| | N1I3 Nivolumab-1/ Ipilimumab-3 (n = 26) | N3I1 Nivolumab-3/ Ipilimumab-1 (n = 104) | N3 Nivolumab-3 (n = 78) |
|---|---|---|---|
| Objective response rate (ORR) | 38.5% | 26.0% | 25.6% |
| Frequency of drug-related grade 3-4 AE | 30.8% | 31.7% | 23.1% |
| Discontinuation | 7.7% | 13.5% | 3.8% |

Second line treatment with 1 mg/kg nivolumab combined with 3 mg/kg ipilimumab (N1I3) may provide the most favorable benefit-risk ratio among the regimens studied. The present study is ongoing.

The foregoing description of the specific embodiments will so fully reveal the general nature of the invention that others can, by applying knowledge within the skill of the art, readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

The following numbered clauses, describing aspects of the invention, are part of the description:
1. A method for treating a subject afflicted with a urothelial carcinoma (UC) or cancer derived therefrom comprising administering to the subject an antibody or an antigen-binding portion thereof that binds specifically to a Programmed Death-1 (PD-1) receptor and inhibits PD-1 activity ("anti-PD-1 antibody").
2. The method of clause 1, further comprising administering to the subject an antibody or an antigen-binding portion thereof that binds specifically to Cytotoxic T-Lymphocyte Antigen-4 (CTLA-4) and inhibits CTLA-4 activity ("anti-CTLA-4 antibody").
3. The method of clause 1 or 2, wherein the UC comprises a bladder cancer, a carcinoma of the ureter, a carcinoma of the renal pelvis, or any combination thereof.
4. The method of any one of clauses 1-3, wherein the UC comprises a transitional cell carcinoma, a squamous cell carcinoma, an adenocarcinoma, or any combination thereof.
5. The method of any one of clauses 1-4, wherein the UC is a recurrent UC.
6. The method of any one of clauses 1-5, wherein the UC is locally advanced or metastatic.
7. The method of any one of clauses 1-6, wherein the subject received at least one, at least two, at least three, at least four, or at least five previous lines of therapy to treat the UC.
8. The method of any one of clauses 1-7, wherein the anti-PD-1 antibody is nivolumab.
9. The method of any one of clauses 2-8, wherein the anti-CTLA-4 antibody is ipilimumab.
10. The method of any one of clauses 1-9, wherein the anti-PD-1 antibody is administered at a dose ranging from at least about 0.1 mg/kg to at least about 10.0 mg/kg body weight once about every 1, 2, 3, or 4 weeks.
11. The method of any one of clauses 2-10, wherein the anti-CTLA-4 antibody is administered at a dose ranging from at least about 0.1 mg/kg to at least about 10.0 mg/kg body weight once about every 1, 2, 3, or 4 weeks.
12. The method of any one of clauses 2-11, wherein:
   (a) the anti-PD-1 antibody is administered at a dose of about 3 mg/kg body weight once about every 3 weeks and the anti-CTLA-4 antibody or antigen-binding portion thereof is administered at a dose of about 1 mg/kg body weight once about every 3 weeks; or
   (b) the anti-PD-1 antibody is administered at a dose of about 1 mg/kg body weight once about every 3 weeks and the anti-CTLA-4 antibody or antigen-binding portion thereof is administered at a dose of about 3 mg/kg body weight once about every 3 weeks.
13. The method of any one of clauses 1-12, wherein the subject has a tumor that has ≥ 1% PD-L1 expression.
14. A kit for treating a subject afflicted with a UC or a cancer derived therefrom, the kit comprising:
   (a) an amount ranging from about 4 mg to about 500 mg of an anti-PD-1 antibody or an antigen-binding portion thereof; and
   (b) instructions for using the PD-1 antibody in the method of any of clauses 1-58.
15. A kit for treating a subject afflicted with a UC or a cancer derived therefrom, the kit comprising:
   (a) an amount ranging from about 4 mg to about 500 mg of an anti-PD-1 antibody or an antigen-binding portion thereof;
   (b) an amount ranging from about 4 mg to about 500 mg of a CTLA-4 antibody or an antigen-binding portion thereof; and
   (c) instructions for using the PD-1 antibody and the CTLA-4 antibody in the method of any of clauses 2-58.

## Claims

1. An antibody or an antigen-binding portion thereof that binds specifically to a Programmed Death-1 (PD-1) receptor and inhibits PD-1 activity ("anti-PD-1 antibody") for use in a method of treating a urothelial carcinoma (UC) in a subject, wherein the subject is administered a combination of the anti-PD-1 antibody and an antibody or an antigen-binding portion thereof that binds specifically to Cytotoxic T-Lymphocyte Antigen-4 (CTLA-4) and inhibits CTLA-4 activity ("anti-CTLA-4 antibody"), wherein the anti-PD-1 antibody comprises nivolumab and the anti-CTLA-4 antibody comprises ipilimumab, and wherein the nivolumab is administered at a dose of 1 mg/kg body weight once every 3 weeks and the ipilimumab or antigen-binding portion thereof is administered at a dose of 3 mg/kg body weight once every 3 weeks.

2. An antibody or an antigen-binding portion thereof that binds specifically to Cytotoxic T-Lymphocyte Antigen-4 (CTLA-4) and inhibits CTLA-4 activity ("anti-CTLA-4 antibody") for use in a method of treating a urothelial carcinoma (UC) in a subject, wherein the subject is administered a combination of the anti-CTLA-4 antibody and an antibody or an antigen-binding portion thereof that binds specifically to a Programmed Death-1 (PD-1) receptor and inhibits PD-1 activity ("anti-PD-1 antibody"), wherein the anti-PD-1 antibody comprises nivolumab and the anti-CTLA-4 antibody comprises ipilimumab, and wherein the nivolumab is administered at a dose of 1 mg/kg body weight once every 3 weeks and the ipilimumab or antigen-binding portion thereof is administered at a dose of 3 mg/kg body weight once every 3 weeks.

3. The antibody for use of claim 1 or 2, wherein the UC comprises a bladder cancer, a carcinoma of the ureter, a carcinoma of the renal pelvis, or any combination thereof.

4. The antibody for use of claim 3, wherein the UC comprises a bladder cancer.

5. The antibody for use of any one of claims 1-4, wherein the UC comprises a transitional cell carcinoma, a squamous cell carcinoma, an adenocarcinoma, or any combination thereof.

6. The antibody for use of any one of claims 1-5, wherein the UC is a recurrent UC.

7. The antibody for use for use of any one of claims 1-6, wherein the UC is locally advanced or metastatic.

8. The antibody for use of any one of claims 1-7, wherein the subject received at least one previous line of therapy to treat the UC.

9. The antibody for use of any one of claims 1-8, wherein the subject has a tumor that has ≥ 1% PD-L1 expression.

10. A kit for use in a method of treating a subject afflicted with a UC according to any one of claims 1 to 9, the kit comprising:
(a) an amount ranging from 4 mg to 500 mg of nivolumab or an antigen-binding portion thereof; and
(b) an amount ranging from 4 mg to 500 mg of ipilimumab or an antigen-binding portion thereof.
